# FASCICULE DE BREVET EUROPEEN

(11) **EP 2 978 459 B1**
(45) Date de publication et mention de la délivrance du brevet: **12.04.2017**
(21) Numéro de dépôt: 14717068.2
(22) Date de dépôt: 27.03.2014
(51) Int. Cl.: A61L 2/22, A61L 2/23, A01N 25/04, A01N 59/00

(54) **GEL ALCALIN OXYDANT DE DÉCONTAMINATION BIOLOGIQUE ET PROCÉDÉ DE DÉCONTAMINATION BIOLOGIQUE DE SURFACES UTILISANT CE GEL.**
OXIDIERENDES ALKALI-BIODEKONTAMINATIONSGEL UND OBERFLÄCHENBIODEKONTAMINATIONSVERFAHREN MIT DIESEM GEL
OXIDIZING ALKALINE BIODECONTAMINATION GEL AND SURFACE BIODECONTAMINATION METHOD USING SAID GEL

(30) Priorité: 29.03.2013 FR 1352907
(43) Date de publication de la demande: 03.02.2016
(73) Titulaire: Commissariat à l'Énergie Atomique et aux Énergies Alternatives, 75015 Paris (FR)
(72) Inventeur: LUDWIG, Amélie, F-13300 Salon De Provence (FR); GOETTMANN, Frédéric, F-84350 Corthezon (FR); FRANCES, Fabien, F-30340 Rousson (FR); LEGOFF, Charline, F-34280 La Grande Motte (FR); TANCHOU, Valérie, F-84100 Orange (FR)
(74) Mandataire: Brevalex
(86) Numéro de dépôt international: PCT/EP2014/056183
(87) Numéro de publication internationale: WO 2014/154818

(56) Documents cités:
- WO-A1-2004/108170
- WO-A1-2012/001046
- WO-A2-2010/079487
- US-A1- 2006 241 002
- US-B1- 6 455 751

## Description

### DOMAINE TECHNIQUE

La présente invention a pour objet un gel alcalin oxydant de décontamination biologique utilisable pour la décontamination de surfaces.

La présente invention a trait, en outre, à un procédé de décontamination biologique de surfaces utilisant ce gel.

L'invention s'applique à la décontamination de surfaces polluées, contaminées, par des agents biologiques.

Le procédé selon l'invention peut s'appliquer à toutes sortes de surfaces telles que les surfaces métalliques, les surfaces en matières plastiques, les surfaces en matériaux vitreux, les surfaces en matériaux cimentaires comme les pâtes, les mortiers et les bétons ; les surfaces en briques ; les surfaces en plâtre ; les surfaces en céramiques ; et les surfaces en pierre naturelle ou artificielle. Ces surfaces peuvent être peintes ou non.

Le domaine technique de l'invention est celui de la décontamination biologique de surfaces contaminées notamment par des espèces biologiques et notamment des espèces biologiques toxiques, par exemple de type endospores, toxines, virus, en vue d'éliminer ces espèces dont la présence sur ces surfaces n'est pas souhaitée, de ces surfaces.

### ÉTAT DE LA TECHNIQUE ANTÉRIEURE

Depuis quelques dizaines d'années, la succession d'actes terroristes chimiques et plus récemment biologiques, par exemple l'attentat au gaz sarin dans le métro de Tokyo en 1995 ou encore l'anthrax dans les courriers piégés de l'US Postal aux Etats-Unis en 2001, a incité de nombreux pays à développer des moyens stratégiques, dits d'intervention post-événementielle, pour répondre efficacement aux conséquences d'éventuelles attaques terroristes employant des agents biologiques, chimiques ou radiologiques.

Essentiellement de nature chimique au début du XX^{ième} siècle, les agents de la menace ont évolué vers des armes de plus forts impacts, plus simples à mettre en oeuvre et surtout non détectables avant l'apparition des premiers symptômes sur l'organisme.

La crainte se porte donc aujourd'hui sur les attaques terroristes de type biologiques, notamment à l'aide d'agents biologiques de classe A, qui sont facilement disséminables, contagieux, et qui causent une morbidité et une mortalité importantes. Les espèces biologiques pathogènes telles que le *Bacillus anthracis* (maladie du charbon ou anthrax) ou encore la bactérie *Yersinia pestis* (peste) sont considérées comme les agents dont la probabilité d'utilisation est la plus forte.

Dans l'hypothèse d'un tel évènement, la priorité pour les autorités est de limiter les effets de l'attaque sur la population civile en décontaminant rapidement les infrastructures exposées, notamment civiles, afin d'éviter une propagation des espèces toxiques par les installations et équipements techniques, tels que les conduits d'aération et les conduits d'évacuation des eaux usées, et de restituer au plus vite les bâtiments à leur usage sans qu'aucun risque d'exposition aux espèces toxiques ne persiste.

Cette décontamination peut passer par deux étapes successives :
- la neutralisation, voire la destruction de l'espèce toxique, lorsque celle-ci est possible.
- le transfert de l'espèce toxique vers une phase solide ou liquide permettant son élimination.

D'une façon générale, les techniques d'assainissement des matériaux contaminés par une contamination biologique consistent en la mise en contact d'un liquide contenant un agent biocide avec les surfaces contaminées. L'application de la solution biocide est généralement réalisée par pulvérisation ou par lavage couplé ou non à un effet mécanique tel qu'un brossage.

Un aperçu de ces techniques est fourni dans les documents FR-A1-2962046 et WO-A1-2012/001046 [1].

Il y est notamment indiqué que les produits de décontamination, qui se présentent sous la forme d'un gel, génèrent un déchet solide et permettent ainsi de s'affranchir de l'utilisation de solutions liquides pour assainir des pièces de grandes surfaces et de géométries complexes.

Ces gels sont généralement mis en oeuvre en les pulvérisant sur la surface à décontaminer.

Après un temps de contact du gel avec la surface à décontaminer, équivalent à la durée d'évaporation du solvant, le déchet sec obtenu est éliminé par brossage et/ou aspiration. L'intérêt majeur de ces procédés est leur aptitude au traitement des grandes surfaces et de géométries accidentées.

Ainsi, le document [2] décrit une composition de gel contenant des agents oxydants pour la décontamination chimique ou biologique de zones contaminées. Cette composition est préparée en ajoutant des agents épaississants ou gélifiants sous la forme de colloïdes à une solution d'agent oxydant pour former un gel colloïdal visqueux.

Cette solution peut être une solution aqueuse ou organique.

Les agents épaississants ou gélifiants peuvent être choisis parmi la silice, l'alumine, les aluminosilicates, les mélanges de silice et d'alumine, et les argiles telles que la smectite.

Les agents oxydants sont notamment l'hypochlorite de sodium, le persulfate d'ammonium, ou le peroxyde d'hydrogène.

Il est mentionné que le gel peut être basique avec un pH supérieur ou égal à 12, mais aucune précision n'est apportée quant à la nature de la base ajoutée pour obtenir un tel pH.

Il est indiqué que ces gels peuvent être utilisés pour éliminer des agents biologiques tels que des micro-organismes comme les bactéries, champignons, virus, et spores, ou des agents chimiques tels que les gaz neurotoxiques.

Les gels sont ensuite pulvérisés sur les surfaces à traiter puis récupérés par aspiration après séchage.

Il est précisé qu'un gel oxydant contenant du peroxymonosulfate de potassium et 15% de silice Cab-O-Sil^{®} EH-5 en tant qu'agent gélifiant, détruit les agents chimiques « *Mustard », « VX* » et « *GD* » dans le temps nécessaire pour amener le gel à siccité et que le *Bacillus globigii* (BG), simulant de l'Anthrax est également détruit en partie par ce gel.

Les formulations gélifiées développées par le Lawrence Livermore National Laboratory sous les noms de L-Gels tels que le L-Gel 115, et L-Gel 200 sont analogues aux formulations développées dans le document [2] et sont mises en oeuvre avec le procédé dit « L-Gel ». Ce procédé semble avoir une certaine efficacité vis-à-vis d'une contamination biologique telle qu'une contamination par les spores de *Bacillus globigii* [3].

Ces gels dits « L-gels » sont formulés à partir de solutions acides oxydantes auxquelles sont ajoutés des solvants organiques et une charge de silice. Les gels sont ensuite pulvérisés sur les surfaces à traiter puis récupérés par aspiration après séchage. Parmi les points critiques de ce procédé, apparaît en premier lieu la présence d'agents oxydants puissants dont la stabilité chimique est souvent très limitée dans le temps.

Par ailleurs, afin d'éviter les coulures, en particulier lorsque le gel (à savoir le gel du document [2] ou le « L-gel ») est appliqué sur les murs ou les plafonds, celui ci est appliqué sous forme de films très minces d'une épaisseur ne dépassant pas, dans le document [2], 125 µm. Il en résulte un déchet sec pulvérulent pouvant entraîner, si l'efficacité du traitement n'est pas totale, une dissémination des espèces bio-toxiques et chimiques, telles que les composés oxydants, dans l'atmosphère.

Les performances du procédé, déterminées vis-à-vis d'une contamination par les spores d'anthrax sous forme d'aérosol (10⁷ et 10⁸ spores par échantillon de 0,16 m²), montrent qu'il n'autorise pas une réduction de la contamination supérieure à 4 décades [3].

Par ailleurs, dans le cadre de la décontamination nucléaire, des formulations gélifiées qui permettent de s'affranchir des problèmes liés au caractère pulvérulent du déchet sec, et d'accroître l'efficacité du procédé mettant en oeuvre un gel ont fait l'objet des documents [4] et [5].

Ces documents décrivent des gels colloïdaux inorganiques dits « gels aspirables », spécifiquement formulés pour être pulvérisés, puis pour sécher en se fracturant, tout en piégeant et confinant la contamination radioactive sous forme de paillettes non-pulvérulentes, aspirables, et directement conditionnables et stockables.

Le document [4] décrit un gel constitué d'une solution colloïdale comprenant un agent viscosant inorganique, généralement de la silice ou de l'alumine, un agent actif de traitement qui est par exemple un acide ou une base inorganique telle que la soude ou la potasse, et éventuellement un agent oxydant ayant un potentiel normal d'oxydoréduction E₀ supérieur à 1,4 V en milieu acide fort tel que Ce(IV), Co(III), ou Ag(II).

Le document [5] décrit un gel constitué d'une solution colloïdale comprenant un agent viscosant organique, généralement de la silice ou de l'alumine, un tensio-actif, un acide ou une base inorganique, éventuellement un agent oxydant ayant un potentiel normal d'oxydoréduction E₀ supérieur à 1,4 V en milieu acide fort tel que Ce(IV), Co(III), ou Ag(II).

Ces gels colloïdaux inorganiques, du fait des différents constituants entrant dans leur composition ont une rhéologie qui permet leur pulvérisation sur une surface contaminée, puis leur adhésion à cette surface, même verticale, sans couler.

Cela permet ainsi un contact prolongé entre le contaminant et l'agent actif de décontamination, sans que les propriétés mécaniques du substrat ne soient altérées.

Suite à sa pulvérisation, le gel sèche, se fracture, et produit des résidus secs, appelés « paillettes », adhérant au substrat et qui sont par la suite évacués par brossage ou aspiration pour être directement conditionnés.

Les procédés de décontamination qui mettent en oeuvre ces gels aspirables sont donc des procédés de décontamination par voie sèche, ne générant aucun effluent liquide et peu de résidus solides secs. En effet, ces résidus solides secs ne représentent en moyenne qu'un quart de la masse de gel initialement pulvérisée. De plus, ces procédés limitent le temps d'exposition des opérateurs à la contamination radioactive, du fait de leur mise en oeuvre facile par pulvérisation puis aspiration des résidus secs, et du fait que la présence de l'opérateur n'est pas requise pendant le séchage du gel.

Les gels décrits dans les documents [4] et [5] sont cependant spécifiquement destinés à la décontamination radioactive de surfaces notamment dans le cadre du démantèlement d'installations nucléaires et ne sont, en aucune manière, adaptés ou susceptibles d'être adaptés à la décontamination biologique de surfaces.

Les documents FR-A1-2962046 et WO-A1-2012/001046 [1] concernent un gel de décontamination biologique « aspirable » et un procédé de décontamination biologique de surfaces utilisant ce gel.

Ce gel est constitué par une solution colloïdale comprenant au moins un agent viscosant inorganique, au moins un agent de décontamination biologique, au moins un polymère super-absorbant, et au moins un agent tensio-actif.

Le polymère super-absorbant, tel que le polyacrylate de sodium, permet d'améliorer l'efficacité du gel sur les matériaux poreux, par exemple les mortiers.

Cependant, ce gel et notamment les gels décrits dans les exemples de ce document qui comprennent de l'alumine, de la soude, un tensio-actif et un polymère super-absorbant qui est un polyacrylate de sodium, n'est pas suffisamment efficace en vue d'une commercialisation dans le domaine de la décontamination NRBC qui requiert une décontamination biologique d'au moins 6 log, et plus exactement comprise entre 6 et 8 log.

Le polymère super-absorbant, tel que le polyacrylate de sodium, permet d'améliorer l'efficacité du gel sur les matériaux poreux, par exemple les mortiers.

Cependant, il a été mis en évidence que le gel de ce document a une durée de conservation très courte, par exemple de quelques semaines.

Cette durée de conservation réduite est particulièrement gênante lorsque le gel est utilisé pour une décontamination NRBC. En effet pour une telle utilisation, le gel doit pouvoir être stocké pendant une durée de plusieurs mois, pouvant même aller jusqu'à 3 ans, et doit pouvoir être directement disponible en cas d'intervention post-évènementielle.

Il existe donc, au regard de ce qui précède, un besoin pour un gel de décontamination biologique dans lequel l'efficacité de l'agent actif de décontamination biologique soit améliorée, en d'autres termes dont l'activité biocide soit renforcée, par rapport aux gels de décontamination de l'art antérieur, et dont la stabilité dans le temps et la durée de conservation soient accrues notamment par rapport au gel décrit dans le document [1].

Il existe notamment un besoin pour un gel de décontamination biologique dont la durée de conservation soit suffisamment longue pour qu'elle permette son utilisation pour une décontamination NRBC et dont les propriétés demeurent intactes même après un stockage de longue durée afin que le gel soit immédiatement disponible en cas d'une intervention « post-évènementielle ».

Ces améliorations en termes d'efficacité de l'agent actif, de stabilité et de durée de conservation, doivent être obtenues sans que les autres propriétés physico-chimiques du gel telles que sa rhéologie ou autres ne soient affectées. En particulier, le gel doit posséder toutes les propriétés d'un gel aspirable avec tous les avantages liés à la mise oeuvre d'un tel gel dans un procédé de décontamination qui ont déjà été exposés plus haut.

Ce gel de décontamination biologique doit produire des déchets secs, non-pulvérulents, faciles à éliminer sans dissémination des contaminants biologiques, permettre de traiter avec la même efficacité une grande variété de surfaces quelles que soient leur forme, leur géométrie, leur taille et leur nature.

De plus, ce gel, au vu de son utilisation finale, ne doit produire aucune altération chimique, mécanique ou physique des surfaces traitées.

Le but de la présente invention est de fournir un gel de décontamination biologique qui réponde entre autres aux besoins et exigences énumérés plus haut.

Le but de la présente invention est encore de fournir un gel de décontamination qui ne présente pas les inconvénients, défauts, limitations et désavantages des gels de décontamination biologique de l'art antérieur et qui résolve les problèmes des gels de décontamination biologique de l'art antérieur, notamment du gel faisant l'objet du document [1].

### EXPOSÉ DE L'INVENTION

Ce but, et d'autres encore, sont atteints, conformément à l'invention, par un gel de décontamination biologique, constitué par une solution colloïdale comprenant, de préférence constitué par :
- 5% à 30% en masse, de préférence 5% à 25% en masse, de préférence encore 8% à 20% en masse par rapport à la masse du gel, d'au moins un agent viscosant inorganique ;
- un agent actif de décontamination biologique constitué par la combinaison d'une base minérale choisie parmi les hydroxydes de métaux alcalins, les hydroxydes de métaux alcalinoterreux, et leurs mélanges, et d'un agent oxydant stable en milieu basique choisi parmi les permanganates, les persulfates, l'ozone, les hypochlorites, et leurs mélanges; la base minérale étant présente à raison de 0,05 à 10 mol/L de gel, de préférence à raison de 0,1 à 5 mol/L de gel, et l'agent oxydant stable en milieu basique étant présent à raison de 0,05 à 5 mol/L de gel, de préférence de 0,1 à 2 mol/L de gel ;
- éventuellement 0,1% à 2% en masse par rapport à la masse du gel, d'au moins un agent tensio-actif;
- et le reste de solvant ;
et le gel ne contenant pas de polymère super-absorbant.

Par « reste de solvant », on entend que le solvant est toujours présent dans la solution colloïdale et que la quantité de solvant est une quantité telle que, lorsqu'elle est ajoutée aux quantités des composants de la solution colloïdale autres que le solvant (que ces composants soient des composants obligatoires ou éventuels cités plus haut, ou encore d'autres composants additionnels optionnels cités, comme les pigments, ou non cités), la quantité totale de tous les composants de la solution colloïdale est de 100% en masse.

Les gels selon l'invention n'ont jamais été décrits dans l'art antérieur.

Le gel selon l'invention, selon une première caractéristique fondamentale, est tout d'abord défini par le fait qu'il contient un agent actif de décontamination biologique constitué par une combinaison spécifique, à savoir la combinaison d'une base minérale spécifique choisie parmi les hydroxydes de métaux alcalins, les hydroxydes de métaux alcalinoterreux, et leurs mélanges, et d'un agent oxydant biocide spécifique qui est un agent oxydant stable en milieu basique choisi parmi les permanganates, persulfates, l'ozone, les hypochlorites, et leurs mélanges.

Un tel agent actif de décontamination biologique constitué par une telle combinaison spécifique, n'est ni décrit ni suggéré dans l'art antérieur.

Le gel selon l'invention est ensuite défini par le fait qu'il ne contient pas de polymère super-absorbant.

*A fortiori,* un gel de décontamination biologique comprenant un agent actif de décontamination biologique spécifique constitué par ladite combinaison d'une base minérale spécifique et d'un agent oxydant spécifique stable en milieu basique n'est ni décrit ni suggéré dans l'art antérieur.

Le gel selon l'invention qui contient un agent actif de décontamination biologique spécifique constitué d'une combinaison d'une base minérale spécifique tel qu'un hydroxyde de métal alcalin, comme la soude, ou un hydroxyde de métal alcalinoterreux, et d'un agent oxydant spécifique tel qu'un hypochlorite, comme l'hypochlorite de sodium, présente, de manière surprenante, une activité biocide renforcée notamment par rapport à des gels, tels que ceux du document [1] contenant un agent actif de décontamination biologique constitué seulement par une base minérale telle que la soude.

On peut dire que l'association d'une base minérale spécifique telle qu'un hydroxyde alcalin, comme la soude, ou un hydroxyde de métal alcalinoterreux et d'un agent oxydant spécifique tel qu'un hypochlorite, comme l'hypochlorite de sodium qui possède lui aussi une activité biocide constitue une véritable combinaison synergique, comme on l'explique plus bas.

En effet, l'agent actif de décontamination biologique du gel selon l'invention comprend bien deux composés actifs de décontamination biologique, deux composés biocides, à savoir un premier composé actif biocide qui est une base minérale telle que la soude et un deuxième composé actif biocide qui est un agent oxydant tel que la javel. C'est cette combinaison des deux composés actifs qui rend le gel encore plus efficace.

L'agent oxydant tel que la javel n'est pas qu'une simple espèce oxydante, c'est également un excellent biocide.

De manière encore plus étonnante, le gel selon l'invention qui possède donc une activité biologique accrue, est cependant aussi stable, et présente une stabilité dans le temps accrue.

En effet, les inventeurs ont mis en évidence que la mauvaise stabilité dans le temps du gel de décontamination biologique du document [1] était due au polymère super-absorbant car ce polymère super-absorbant modifie la rhéologie du gel lors de son stockage, ce qui le rend impropre à la pulvérisation et à l'application sur une surface verticale, du fait d'une mauvaise adhérence.

Les inventeurs ont, en outre, mis en évidence que l'utilisation d'agents oxydants en présence de polymères super-absorbants réduisait encore considérablement la stabilité dans le temps du gel de décontamination biologique du document [1], jusqu'à une durée inférieure à quelques jours (voir les exemples).

L'absence de polymère super-absorbant dans le gel selon l'invention en améliore donc grandement la stabilité dans le temps.

Le gel selon l'invention constitue donc une amélioration considérable de la formulation des gels de décontamination biologique de l'art antérieur et notamment du gel faisant l'objet du document [1], aussi bien du point de vue de son efficacité biocide que de sa stabilité dans le temps.

On peut dire que dans le gel de décontamination biologique selon l'invention, d'une part on améliore l'efficacité de l'actif de décontamination, et d'autre part on accroît la stabilité du gel en s'affranchissant de l'addition d'un polymère super-absorbant.

Plus exactement, de façon tout à fait surprenante, et contrairement à ce à quoi l'on pouvait s'attendre au regard des résultats obtenus avec le gel de décontamination biologique du document [1], le gel selon l'invention présente une activité biocide par exemple supérieure de 2 à 3 ordres de grandeur par rapport au gel du document [1], sans toutefois s'altérer dans le temps, à savoir sur une durée par exemple de : voir les exemples.

De préférence, la base minérale est choisie parmi l'hydroxyde de sodium, l'hydroxyde de potassium, et leurs mélanges, et l'agent oxydant stable en milieu basique est choisi parmi les hypochlorites, et leurs mélanges.

Un agent actif de décontamination biologique particulièrement préféré est constitué par la combinaison de soude et d'hypochlorite de sodium.

Dans ce cas, la soude est présente à raison de 0,05 à 10 mol/L de gel, de préférence 0,5 à 5 mol/L de gel, et l'hypochlorite de sodium est présent à raison de 0,05 à 5 mol/L de gel, de préférence de 0,1 à 1,5 mol/L de gel.

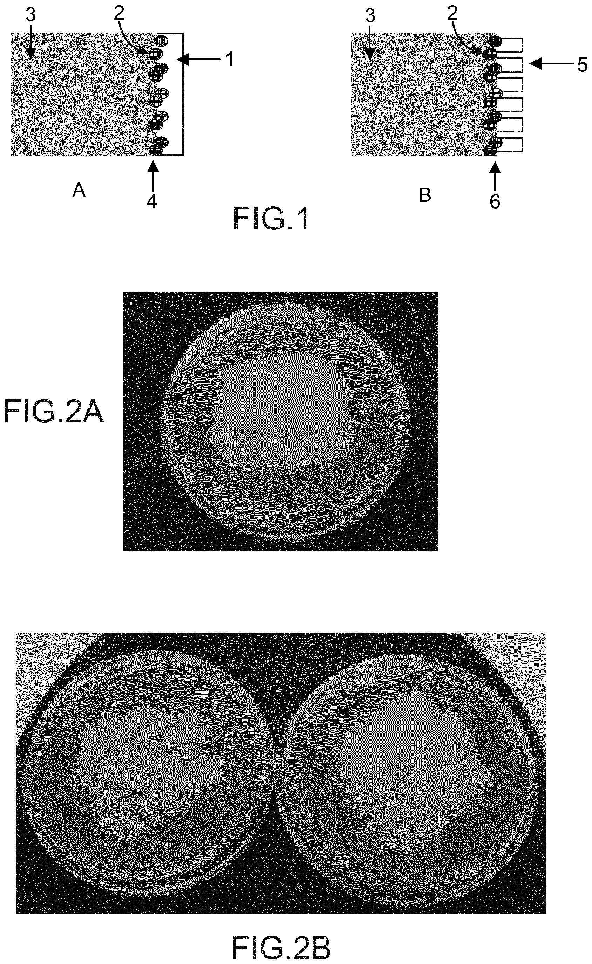

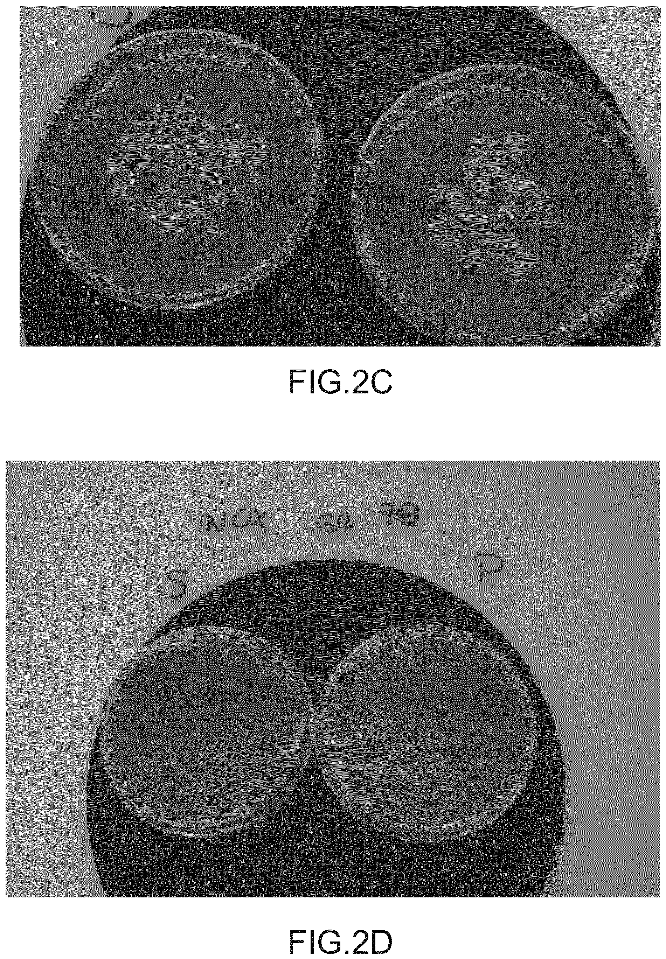

En effet, l'addition d'hypochlorite de sodium (concentré de Javel) permet de renforcer l'agressivité biocide du gel selon l'invention et donc d'en augmenter le facteur de décontamination biologique par rapport à un gel ne contenant que de la soude (Figure 2) sans en modifier fondamentalement les propriétés physico-chimiques ou la rhéologie. La soude, est quant à elle également un bon biocide. De plus c'est un excellent stabilisant de l'hypochlorite de sodium, et elle garantit une bonne conservation de la teneur en ion hypochlorite tout en assurant une fonction biocide.

En résumé, les gels selon l'invention répondent donc à l'ensemble des besoins mentionnés plus haut, ils ne présentent pas les inconvénients, défauts, limitations et désavantages des gels de décontamination biologique de l'art antérieur, tels que ceux décrits dans les documents mentionnés plus haut.

Les gels selon l'invention résolvent ainsi les problèmes présentés par les gels de décontamination biologique de l'art antérieur sans en présenter les inconvénients mais tout en conservant toutes les propriétés avantageuses connues de ces gels, notamment leur caractère « aspirable ».

Le gel selon l'invention est une solution colloïdale, ce qui signifie que le gel selon l'invention contient des particules solides inorganiques, minérales, d'agent viscosant dont les particules élémentaires, primaires, ont une taille généralement de 2 à 200 nm.

Du fait de la mise en oeuvre d'un agent viscosant généralement exclusivement inorganique, sans agent viscosant organique, la teneur en matières organiques du gel selon l'invention est généralement inférieure à 4% en masse, de préférence inférieure à 2% en masse, ce qui constitue encore un autre avantage des gels selon l'invention.

Ces particules solides, minérales, inorganiques jouent le rôle de viscosant pour permettre à la solution, par exemple la solution aqueuse, de se gélifier et ainsi d'adhérer aux surfaces à traiter, décontaminer, quelles que soient leur géométrie, leur forme, leur taille, et où que se trouvent les contaminants à éliminer.

Avantageusement, l'agent viscosant inorganique peut être choisi parmi les oxydes de métaux tels que les alumines, les oxydes de metalloïdes à l'exception de la silice, les hydroxydes de métaux, les hydroxydes de metalloïdes, les oxyhydroxydes de métaux, les oxyhydroxydes de métalloïdes, les aluminosilicates, les argiles telles que la smectite, et leurs mélanges ; ces agents viscosant sont stables en milieu basique.

En particulier, l'agent viscosant inorganique peut être choisi parmi les alumines (Al₂O₃).

L'agent viscosant inorganique peut ne comprendre qu'une seule alumine ou un mélange de celles-ci, à savoir un mélange de deux alumines, différentes ou plus (mélange Al₂O₃/Al₂O₃).

L'alumine peut être choisie parmi les alumines calcinées, les alumines calcinées broyées, et leurs mélanges.

A titre d'exemple, on peut citer le produit vendu par la société EVONIK INDUSTRIES sous la désignation commerciale « Aeroxide Alu C » qui est de l'alumine fine pyrogénée et qui possède une surface spécifique BET de 100 m²/g.

De manière avantageuse, selon l'invention, l'agent viscosant est constitué par une ou plusieurs alumine(s). Cette ou ces alumine(s) représente(nt) généralement de 5% à 30% en masse par rapport à la masse du gel.

Dans ce cas, le ou les alumine(s) est(sont) de préférence à une concentration de 8% à 17% en masse par rapport à la masse totale du gel (pour assurer un séchage du gel à une température comprise entre 20°C et 50°C et à une humidité relative comprise entre 20% et 60% en moyenne en 30 minutes à 5 heures).

La nature de l'agent viscosant minéral, notamment lorsqu'il est constitué d'une ou plusieurs alumine(s), influence de manière inattendue le séchage du gel selon l'invention et la granulométrie du résidu obtenu.

En effet, le gel sec se présente sous la forme de particules de taille contrôlée, plus précisément de paillettes solides millimétriques, dont la taille va généralement de 1 à 10 mm, de préférence de 2 à 5 mm grâce notamment aux compositions précitées de la présente invention, en particulier lorsque l'agent viscosant est constitué par une ou plusieurs alumine(s).

Précisons que la taille des particules correspond généralement à leur plus grande dimension.

Le gel selon l'invention contient un agent actif de décontamination biologique tel que défini plus haut.

Par agent de décontamination biologique que l'on peut aussi qualifier d'agent biocide, on entend un agent, qui lorsqu'il est mis en contact avec une espèce biologique et notamment une espèce biologique toxique est susceptible, d'inactiver ou de tuer celle-ci.

Par espèce biologique, on entend tout type de micro-organisme tel que les bactéries, les champignons, les levures, les virus, les toxines, les spores notamment les spores de *Bacillus anthracis,* les prions et les protozoaires.

Les espèces biologiques qui sont éliminées, détruites, inactivées, par le gel selon l'invention sont essentiellement des espèces bio-toxiques telles que les spores pathogènes comme par exemple les spores de *Bacillus anthracis,* les bactéries comme par exemple les bactéries *Yersinia pestis,* les toxines comme par exemple la toxine botulique ou la ricine, et les virus comme par exemple le virus de la vaccine ou les virus des fièvres hémorragiques (de type Ebola par exemple).

L'agent actif de décontamination biologique est utilisé aux concentrations mentionnées plus haut, afin de garantir un pouvoir d'élimination des espèces biologiques, notamment biotoxiques, compatible avec le temps de séchage du gel et pour assurer par exemple un séchage du gel à une température comprise entre 20°C et 50°C et à une humidité relative comprise entre 20% et 60 % en moyenne en 30 minutes à 5 heures.

Il est à noter que le gel de l'invention étant un gel basique, il a, outre l'action de décontamination, une action de dégraissage.

De manière à atteindre une efficacité totale, y compris dans les conditions climatiques les plus défavorables vis-à-vis du temps de séchage du gel, le gel selon l'invention peut présenter une large gamme de concentration en agent(s) de décontamination biologique(s) basique(s).

En effet, l'augmentation de la concentration en agent de décontamination biologique basique comme NaOH ou KOH, jouant généralement le rôle d'agent biocide, permet d'accroître considérablement les vitesses de destruction des espèces biologiques, comme par exemple les spores de *Bacillus thuringiensis* (simili des spores de *Bacillus anthracis*).

La base minérale est utilisée à la concentration définie plus haut pour assurer un séchage du gel à température comprise entre 20°C et 50°C et humidité relative comprise entre 20% et 60% en moyenne en 30 minutes à 5 heures.

Dans le cas du traitement d'une matrice cimentaire, le pH basique du gel, qui est induit par exemple par l'utilisation de la soude ou de la potasse, permet d'éviter les réactions acido-basiques, entre le matériau à décontaminer et le gel, qui nuisent à l'intégrité du matériau mais également celle du gel sur la surface et donc à l'efficacité du procédé.

Le caractère hygroscopique de l'hydroxyde de sodium ou de l'hydroxyde de potassium constitue également un atout considérable pour ralentir le phénomène de séchage du gel. Le temps de contact entre le gel selon l'invention, contenant par exemple une solution biocide, et la contamination biologique, s'en retrouve alors considérablement augmenté.

En effet, la compétition entre le processus d'évaporation de la phase aqueuse et celui de reprise d'eau des cristaux d'hydroxyde de sodium ou d'hydroxyde de potassium modifie favorablement la cinétique de séchage du gel.

Conformément à l'invention, le gel selon l'invention ne contient pas, au contraire du gel décrit dans le document [1], de polymère super-absorbant, en d'autres termes le gel selon l'invention est exempt de polymère super-absorbant.

Par « polymère super-absorbant » également dénommé « SAP », on entend généralement un polymère capable, à l'état sec, d'absorber spontanément au moins 10 fois, de préférence au moins 20 fois son poids de liquide aqueux, en particulier d'eau et notamment d'eau distillée. De tels polymères super-absorbants ont été décrits en détail dans le document [1] déjà cité.

Le gel peut aussi contenir, éventuellement, un agent tensio-actif ou un mélange d'agents tensio-actifs, de préférence choisis parmi les agents tensio-actifs non ioniques tels que les copolymères blocs, séquencés comme les copolymères séquencés d'oxyde d'éthylène et d'oxyde de propylène, et les acides gras éthoxylés ; et leurs mélanges.

Pour ce type de gel, les agents tensio-actifs sont de préférence des copolymères blocs commercialisés par la société BASF sous la dénomination "Pluronic^{®}". On pourra utiliser par exemple le Pluronic^{®} PE6200.

Les Pluronics^{®} sont des copolymères séquencés d'oxyde d'éthylène et d'oxyde de propylène.

Ces agents tensio-actifs influencent les propriétés rhéologiques du gel, notamment le caractère thixotropique du produit et le temps de reprise, afin de le rendre pulvérisable aussi bien sur les planchers, les murs ou les plafonds en évitant l'apparition de coulure.

Les tensio-actifs permettent, par ailleurs, de maîtriser l'adhésion du déchet sec et de contrôler la taille des paillettes de résidu sec pour garantir la non-pulvérulence du déchet. Ces tensio-actifs permettent enfin de contrôler le phénomène de ressuage du gel au cours du temps et améliore donc ainsi sa capacité à être pulvérisé après stockage.

Le solvant selon l'invention est généralement choisi parmi l'eau, les solvants organiques, et leurs mélanges.

Un solvant préféré est l'eau, et dans ce cas, le solvant est donc constitué par de l'eau, comprend 100% d'eau.

Avantageusement, le gel selon l'invention peut en outre comprendre au moins un pigment minéral tel que de l'oxyde de fer.

Généralement, la solution colloïdale peut comprendre de 0,01% à 10% en masse, de préférence de 0,1% à 5% en masse par rapport à la masse du gel, dudit au moins un pigment minéral.

Il n'existe aucune limitation quant au pigment minéral qui est incorporé dans le gel de décontamination selon l'invention.

Généralement le pigment minéral est choisi parmi les pigments minéraux qui sont stables dans le gel, notamment eu égard à l'agent actif de décontamination que contient le gel.

Par pigment stable, on entend généralement que le pigment ne présente pas de changement stable de sa couleur dans le temps, lors du stockage du gel pendant une durée minimale de 6 mois.

Il n'y a aucune limitation quant à la couleur de ce pigment, qui est généralement la couleur qu'il va communiquer au gel. Ce pigment peut être de couleur noire, rouge, bleue, verte, jaune, orange, violette, marron, etc., et même blanche.

Généralement, le gel a donc une couleur identique à la couleur du pigment qu'il contient. Il est toutefois possible que le gel possède une couleur qui diffère de la couleur du pigment qu'il contient, par exemple dans le cas où le pigment réagit avec l'actif de décontamination, mais cela n'est pas recherché.

Le pigment, notamment lorsqu'il est blanc, est généralement différent de l'agent viscosant inorganique.

Avantageusement, le pigment minéral est choisi de telle sorte qu'il donne au gel (c'est-à-dire au gel à l'état humide tel que défini plus haut, avant séchage) une couleur différente de la couleur d'une surface à décontaminer sur laquelle le gel est appliqué.

Avantageusement, le pigment minéral est un pigment micronisé, et la taille moyenne des particules du pigment minéral peut être de 0,05 à 5 µm, de préférence de 0,1 à 1 µm.

Le fait que le pigment soit micronisé permet d'éviter qu'il ne modifie la rhéologie et l'aptitude à la pulvérisation du gel (« pulvérisabilité ») car le pigment a alors la même taille micrométrique qui est généralement celle de l'agent viscosant inorganique, tel que les agrégats d'alumine.

Avantageusement, le pigment minéral est choisi parmi les oxydes de métal (métaux) et/ou de métalloïde(s), les hydroxydes de métal (métaux) et/ou de métalloïde(s), les oxyhydroxydes de métal (métaux) et/ou de métalloïde(s), les ferrocyanures et ferricyanures de métal (métaux), les aluminates de métal (métaux), et leurs mélanges.

De préférence, le pigment minéral est choisi parmi les oxydes de fer, de préférence micronisés, et leurs mélanges.

Les oxydes de fer peuvent avoir différentes couleurs, ils peuvent être par exemple jaunes, rouges, violets, oranges, marrons, ou noirs.

En effet, les pigments d'oxyde de fer sont reconnus pour avoir un bon pouvoir couvrant et une grande résistance aux acides et aux bases.

Pour une incorporation dans un gel de décontamination, les oxydes de fer présentent les meilleures performances en termes de stabilité et de pouvoir colorant. Ainsi, une teneur en oxyde de fer de 0,1%, voire 0,01% en masse suffit à fortement colorer le gel sans en modifier les propriétés.

Comme on l'a déjà indiqué plus haut, le fait que le pigment d'oxyde de fer soit de préférence micronisé permet d'éviter qu'il ne modifie la rhéologie et l'aptitude à la pulvérisation du gel (« pulvérisabilité ») car le pigment a alors une taille micrométrique, à savoir une taille qui est généralement celle de l'agent viscosant inorganique, tel que les agrégats d'alumine.

Des oxydes de fers micronisés sont disponibles auprès de la société Rockwood^{®} sous la dénomination commerciale Ferroxide^{®}.

On peut citer entre autres le Ferroxide^{®} 212 M qui est un oxyde de fer rouge micronisé ave une taille moyenne des particules de 0,1 µm et le Ferroxide^{®} 228 M qui est un oxyde de fer rouge micronisé avec une taille moyenne des particules de 0,5 µm.

En plus et/ou à la place des oxydes de fer, d'autres oxydes ou hydroxydes de métaux ou de métalloïdes colorés peuvent être incorporés dans le gel selon l'invention, en fonction du pH du gel, on peut notamment citer l'oxyde de vanadium (V₂O₅) qui est orange, l'oxyde de manganèse (MnO₂) qui est noir, l'oxyde de cobalt qui est bleu ou vert, et les oxydes de terres rares. Cependant les oxydes de fer sont préférés pour les raisons précisées plus haut.

Parmi les oxyhydroxydes, on peut citer la goethite, c'est-à-dire l'oxyhydroxyde de fer FeOOH, qui est très colorée.

A titre d'exemple de ferrocyanure de métal, on peut citer, le bleu de Prusse, c'est-à-dire le ferrocyanure ferrique, et à titre d'exemple d'aluminate, on peut citer le bleu de cobalt, c'est-à-dire l'aluminate de cobalt.

L'incorporation dans le gel selon l'invention d'un pigment minéral permet de mieux visualiser le gel humide puis les résidus secs quel que soit le substrat sur lequel est appliqué le gel.

De manière étonnante, il a été mis en évidence que la substance colorante spécifique qui peut être incorporée dans le gel selon l'invention qui est un pigment minéral, n'affecte pas les propriétés décontaminantes et physico-chimiques du gel de décontamination selon l'invention qui est, à l'instar des gels sans pigments inorganique (minéral), pulvérisable, aspirable après séchage et utilisable dans de nombreuses situations sur une large gamme de contaminants biologiques et de substrats.

Autrement dit, il a été mis en évidence que parmi tous les colorants et pigments qui auraient pu être utilisés pour donner une couleur aux gels de décontamination biologiques selon l'invention projetables et aspirables, seuls les pigments minéraux, plus particulièrement les pigments à base d'oxydes de métal (métaux) et/ou de métalloïde(s), d'hydroxydes de métal (métaux) et/ou de métalloïde(s), d'oxyhydroxydes de métal (métaux) et/ou de métalloïde(s), de ferrocyanures et ferricyanures de métal, d'aluminates de métal (métaux), et de leurs mélanges ; et plus particulièrement encore les pigments à base d'oxydes de fer micronisés, étaient compatibles avec la formulation du gel de décontamination alcalin oxydant selon l'invention, c'est-à-dire n'affectaient en rien les propriétés nécessaires des gels selon l'invention et les avantages qui en découlent.

De façon surprenante, seuls les pigments minéraux, plus particulièrement les pigments à base d'oxydes, d'hydroxydes, d'oxyhydroxydes, de ferrocyanures, de ferricyanures, et d'aluminates ,plus particulièrement encore les pigments à base d'oxydes de fer micronisés, offrent un bon pouvoir colorant et une bonne préservation de la coloration dans le temps sans pour autant modifier de façon notable les propriétés (voir plus haut) du gel alcalin oxydant formulé selon l'invention.

L'addition éventuelle de pigments minéraux au gel selon l'invention permet par de nombreux aspects, de faciliter et d'améliorer sa mise en oeuvre, notamment en ce qui concerne leur utilisation dans des zones sinistrées, en situation d'urgence dans des milieux confinés ou à visibilité réduite, en particulier pour les opérateurs en combinaison NRBC.

La présence éventuelle de pigments minéraux dans le gel selon l'invention assure non seulement une meilleure visualisation des zones recouvertes par le gel humide après pulvérisation mais aussi une meilleure visualisation des paillettes sèches sur le support décontaminé.

Un autre avantage supplémentaire de l'incorporation éventuelle d'un pigment dans le gel selon l'invention est qu'il permet de facilement distinguer les zones sèches, à savoir les zones couvertes par les paillettes de gel sec, des zones de gel encore humides.

Cela est possible grâce à une décoloration du gel au cours du séchage si, bien sûr, le pigment n'est pas un pigment blanc.

On peut ainsi s'assurer visuellement, facilement, et avec certitude que l'action du gel est terminée et que la durée pendant laquelle il est resté sur le substrat a été suffisante pour permettre le séchage complet du gel, alors même que cette durée est aléatoire et varie en fonction des conditions climatiques, à savoir notamment la température, l'humidité relative, et la ventilation.

L'invention concerne, en outre, un procédé de décontamination biologique d'une surface d'un substrat solide contaminée par au moins une espèce biologique se trouvant sur ladite surface, dans lequel on réalise au moins un cycle comprenant les étapes successives suivantes :
a) on applique le gel selon l'invention tel que décrit plus haut sur ladite surface ;
b) on maintient le gel sur la surface au moins pendant une durée suffisante pour que le gel détruise et/ou inactive et/ou absorbe l'espèce biologique, et pour que le gel sèche et forme un résidu sec et solide non pulvérulent contenant éventuellement ladite espèce biologique ;
c) on élimine le résidu sec et solide contenant éventuellement ladite espèce biologique.

Généralement, les résidus solides ne contiennent pas d'espèce biologique vivante.

La contamination biologique détruite, « tuée », est récupérée par les paillettes de gel sec.

Avantageusement, le substrat est en au moins un matériau choisi parmi les métaux et alliages comme l'acier inoxydable ; les aciers peints ; les polymères tels que les matières plastiques ou les caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments ; les mortiers et bétons ; les plâtres ; les briques ; la pierre naturelle ou artificielle ; les céramiques.

Avantageusement, l'espèce biologique est choisie parmi les espèces biologiques toxiques déjà énumérées plus haut.

Avantageusement, le gel est appliqué sur la surface à décontaminer à raison de 100 g à 2000 g de gel par m² de surface, de préférence de 500 à 1500 g de gel par m² de surface, de préférence encore de 600 à 1000 g de gel par m² de surface, ce qui correspond généralement à une épaisseur de gel déposé sur la surface comprise entre 0,5 mm et 2 mm.

Avantageusement, le gel est appliqué sur la surface solide par pulvérisation, au pinceau, ou avec une taloche.

Avantageusement (lors de l'étape b)), le séchage est réalisé à une température de 1°C à 50°C, de préférence de 15°C à 25°C, et sous une humidité relative de 20% à 80%, de préférence de 20% à 70%.

Avantageusement, le gel est maintenu sur la surface pendant une durée de 2 à 72 heures, de préférence de 2 à 48 heures, de préférence encore de 3 à 24 heures.

Avantageusement, le résidu sec et solide se présente sous la forme de particules, par exemple de paillettes, d'une taille de 1 à 10 mm, de préférence de 2 à 5 mm.

Avantageusement, le résidu sec et solide est éliminé de la surface solide par brossage et/ou aspiration.

Avantageusement, le cycle décrit plus haut peut être répété par exemple de 1 à 10 fois en utilisant le même gel lors de tous les cycles ou en utilisant des gels différents lors d'un ou de plusieurs cycle(s).

Avantageusement, lors de l'étape b), le gel, avant séchage total, est remouillé avec une solution d'un agent de décontamination biologique, de préférence avec la solution de l'agent actif biologique du gel appliqué lors de l'étape a) dans le solvant de ce gel.

Lors de l'étape b), le gel peut avant séchage total être remouillé avec la solution biocide contenue dans le gel de décontamination biologique déjà décrit plus haut, ce qui évite alors généralement de répéter l'application du gel sur la surface et occasionne une économie de réactif et une quantité de déchet limitée. Cette opération de remouillage peut être répétée.

En résumé, le procédé et le gel selon l'invention présentent entre autres les propriétés avantageuses suivantes :
- l'application du gel par pulvérisation,
- l'adhérence aux parois,
- l'obtention de l'efficacité maximale de décontamination à l'issue de la phase de séchage du gel.

En général, on fait en sorte que le temps de séchage soit supérieur ou égal à la durée nécessaire pour l'inactivation.
- le traitement par voie sèche d'une gamme très large de matériaux,
- l'absence d'altération mécanique ou physique des matériaux à l'issue du traitement,
- la mise en oeuvre du procédé dans des conditions climatiques variables,
- la réduction du volume de déchet,
- la facilité de récupération du déchet sec,
- la faible exposition des opérateurs à la contamination.

D'autres caractéristiques et avantages de l'invention apparaîtront mieux à la lecture de la description détaillée qui suit, cette description étant faite à titre illustratif et non limitatif, en liaison avec les dessins joints.

### BRÈVE DESCRIPTION DES DESSINS.

- La Figure 1 (A, B) présente des vues en coupe schématique illustrant les étapes principales du procédé selon l'invention pour la décontamination d'un matériau solide.
- La Figure 2 (A, B, C, D) présente des photographies de Boîtes de Pétri dans lesquelles ont été mis en culture des prélèvements issus d'un support en acier inoxydable initialement contaminé par 10⁷ spores de *Bacillus thuringiensis* (*simili* de *Bacillus anthracis,* bactérie responsable de la maladie du charbon ou anthrax) et qui ensuite n'a pas été décontaminé (Figure 2A) ou qui a été décontaminé par un gel inactif, à l'eau (Figure 2B), ou par un gel ancienne formulation (c'est-à-dire le gel GB 69 à la soude et sans PSA) (Figure 2C), ou par un gel GB79 selon l'invention (Figure 2D).

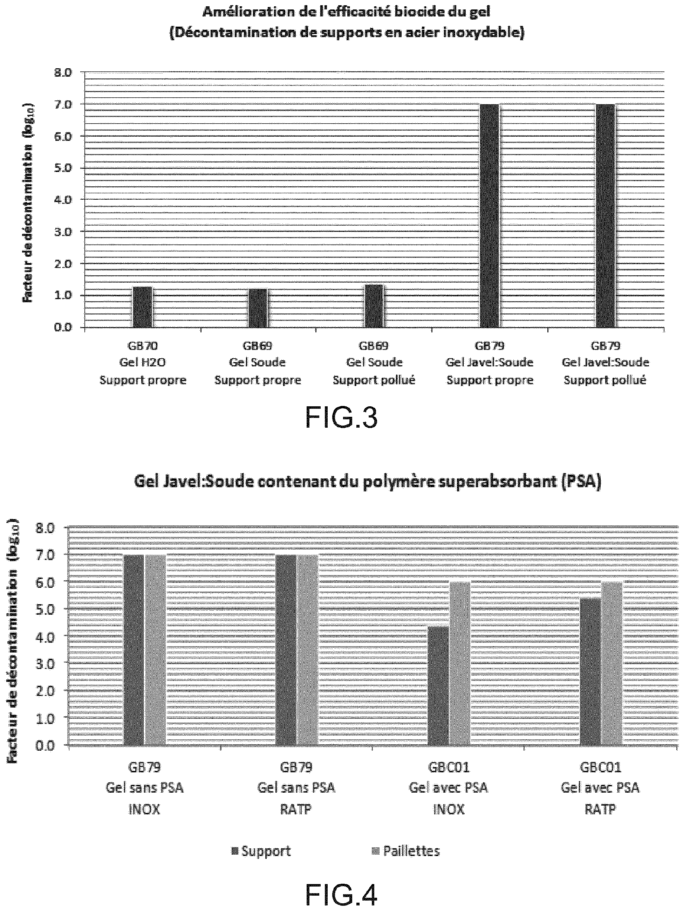

- La Figure 3 est un graphique qui compare l'efficacité biocide, exprimée par le facteur de décontamination (log₁₀), de différents gels sur des supports en acier inoxydable propres ou pollués (par un mélange d'argile Montmorillonite, d'huile de moteur 15W40 et d'éthanol (simili de supports usagés et souillés)), contaminés par des spores de *Bacillus thuringiensis,* à savoir de gauche à droite le gel GB70 qui est un gel inactif à l'eau, sur un support propre ; le gel GB69 qui est un gel à la soude, sur un support propre contaminé; le gel GB69 sur un support pollué contaminé ; le gel GB79 qui est un gel à la soude et à la Javel conforme à l'invention, sur un support propre contaminé ; le gel GB79 qui est un gel à la soude et à la Javel conforme à l'invention, sur un support pollué contaminé.
- La Figure 4 est un graphique qui compare l'efficacité biocide, exprimée par le facteur de décontamination (log₁₀) des gels sans (GB79) et avec polymère superabsorbant (PSA) (GBC01) sur un support en acier inoxydable ou sur un support qui est un carreau en céramique fourni par la RATP, à savoir de gauche à droite le gel GB79 puis le gel GBC01.

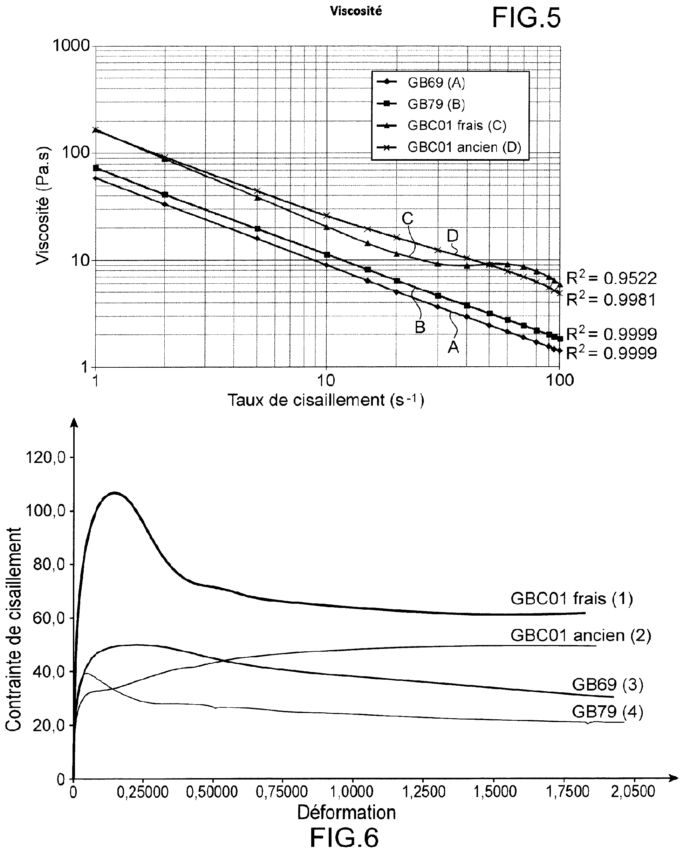

- La Figure 5 est un graphique qui donne la viscosité (en Pa.s) en fonction du taux de cisaillement (en s⁻¹) pour les gels GB69 (◆) (courbe A), GB79 (■) (Courbe B), GBC01 frais, venant d'être préparé, aussi appelé gel neuf (▲) (courbe C), et GBC01 conservé plus d'un mois, aussi appelé gel ancien (x) (courbe D).
- La Figure 6 est un graphique qui donne la contrainte de cisaillement (en Pa) en fonction de la déformation pour le gel GBC01 neuf (courbe 1), le gel GBC01 ancien (courbe 2) ; le gel GB69 (courbe 3) ; et le gel GB79 (courbe 4).

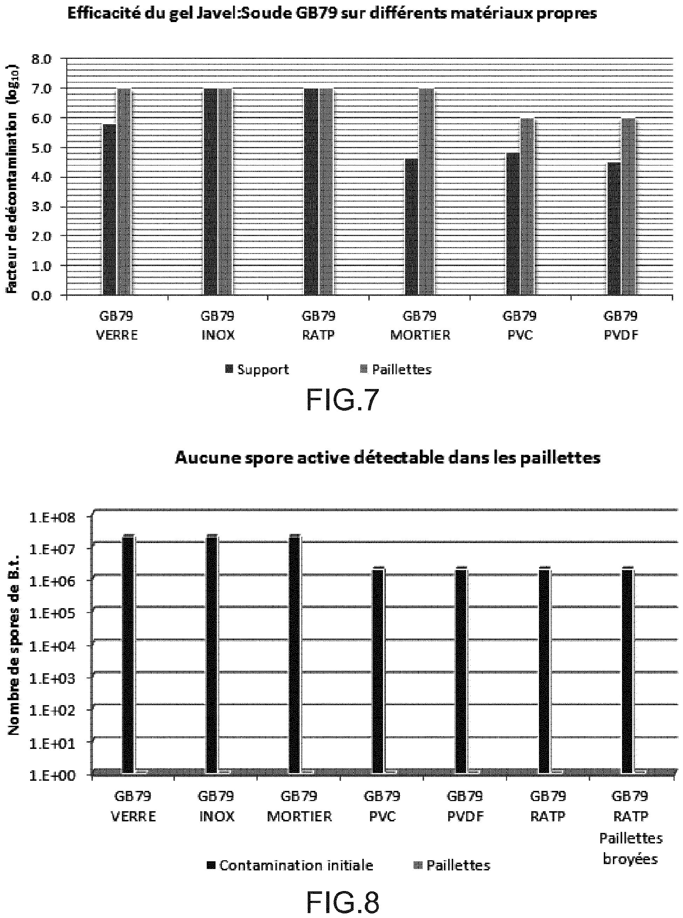

- La Figure 7 est un graphique qui donne l'efficacité biocide, exprimée par le facteur de décontamination (log₁₀), du gel GB79 qui est un gel à la soude et à la Javel conforme à l'invention sur des supports propres en différents matériaux, à savoir de gauche à droite : un support en verre (appelé support VERRE), un support en acier inoxydable (appelé support INOX), un carreau en céramique fourni par la RATP (appelé support RATP), un support en mortier (appelé support MORTIER), un support en PVC (polychlorure de vinyle), et un support en PVDF (polyfluorure de vinylidène). Sur ce graphique pour chaque support, outre le facteur de décontamination obtenu pour le support (barre de gauche), est également porté le facteur de décontamination obtenu dans les résidus secs, paillettes (barre de droite).
- La Figure 8 est un graphique qui donne l'activité biologique détectable, exprimée en nombre de spores de B.t (*Bacillus thuringiensis*), dans les résidus secs, paillettes, obtenus après séchage du gel GB79 qui est un gel à la soude et à la Javel conforme à l'invention sur des supports propres en différents matériaux, à savoir de gauche à droite : un support en verre (appelé support VERRE), un support en acier inoxydable (appelé support INOX), un support en mortier (appelé support MORTIER), un support en PVC (polychlorure de vinyle), un support en PVDF (polyfluorure de vinylidène), un support qui est un carreau en céramique fourni par la RATP, et enfin un support qui est un carreau en céramique fourni par la RATP, les paillettes ayant été finement broyées.

Sur ce graphique pour chaque support est porté le nombre de spores initialement déposées sur le support (contamination initiale) (barre de gauche) et dans les paillettes (non broyées ou broyées dans le dernier cas) (barre de droite).

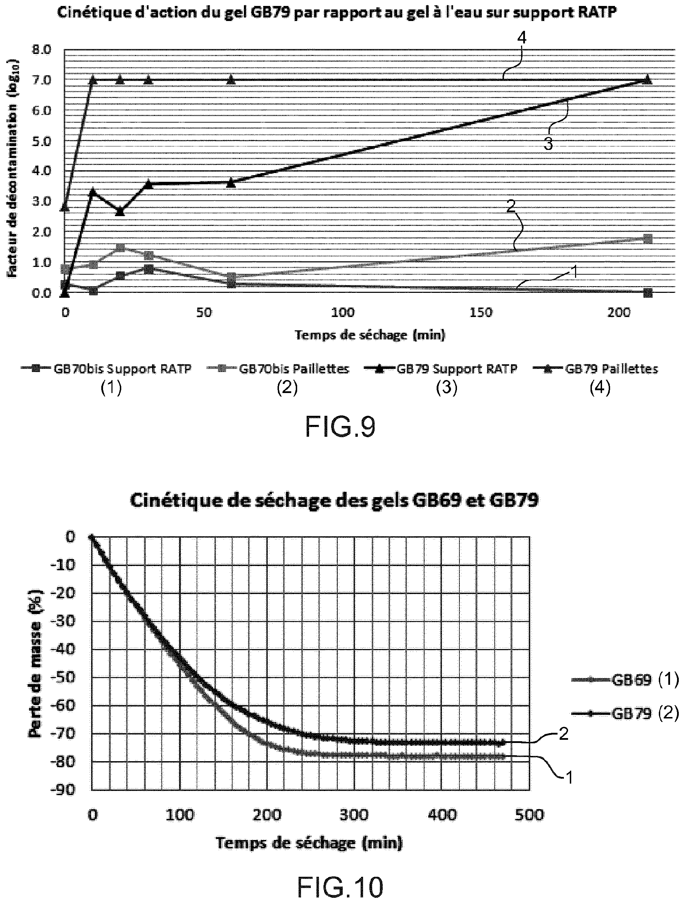

- La Figure 9 est un graphique qui montre la cinétique d'action du gel inactif GB70bis à l'eau et du gel GB79 selon l'invention sur des spores de *Bacillus thuringiensis.* Les gels sont appliqués sur des carreaux en céramique fournis par la RATP.

En abscisse est porté le temps de séchage (en min.) et en ordonnée est porté le facteur de décontamination (log₁₀).

La courbe 1 concerne le gel GB70bis, la courbe 2 concerne les paillettes du gel GB70bis, la courbe 3 concerne le gel GB79, et la courbe 4 concerne les paillettes du gel GB79.
- La Figure 10 est un graphique qui montre la cinétique de séchage, sous atmosphère contrôlée (Température: 25°C ; Humidité relative : 50%; Ouverture de la porte de la balance : 3 cm ; Epaisseur de gel : 0,5 mm), du gel GB69 et du gel GB 79 selon l'invention.

En abscisse est porté le temps de séchage (en min.), et en ordonnée est portée la perte de masse (en %).

La courbe 1 représente la cinétique de séchage du gel GB69 et la courbe 2 représente la cinétique de séchage du gel GB79.

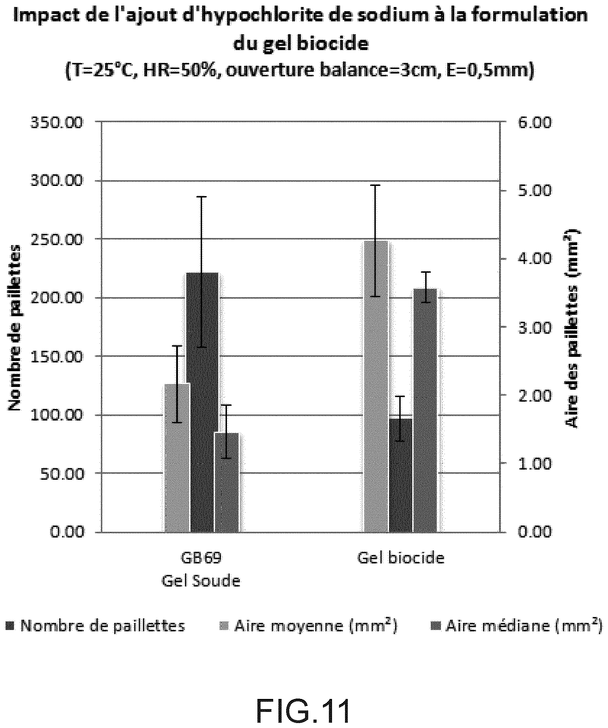

- La Figure 11 est un graphique qui compare la fracturation sous atmosphère contrôlée (Température: 25°C; Humidité relative : 50%; Ouverture de la porte de la balance : 3 cm ; Epaisseur de gel : 0,5 mm) du gel à la soude GB69 et du gel biocide à la Javel et à la soude GB79 selon l'invention (à droite).

L'échelle à gauche indique le nombre de paillettes, et l'échelle à droite indique l'aire des paillettes (en mm²).

Pour chaque gel est porté l'aire moyenne des paillettes (en mm²) (barre de gauche), le nombre de paillettes (barre du milieu), et l'aire médiane (en mm²) (barre de droite).

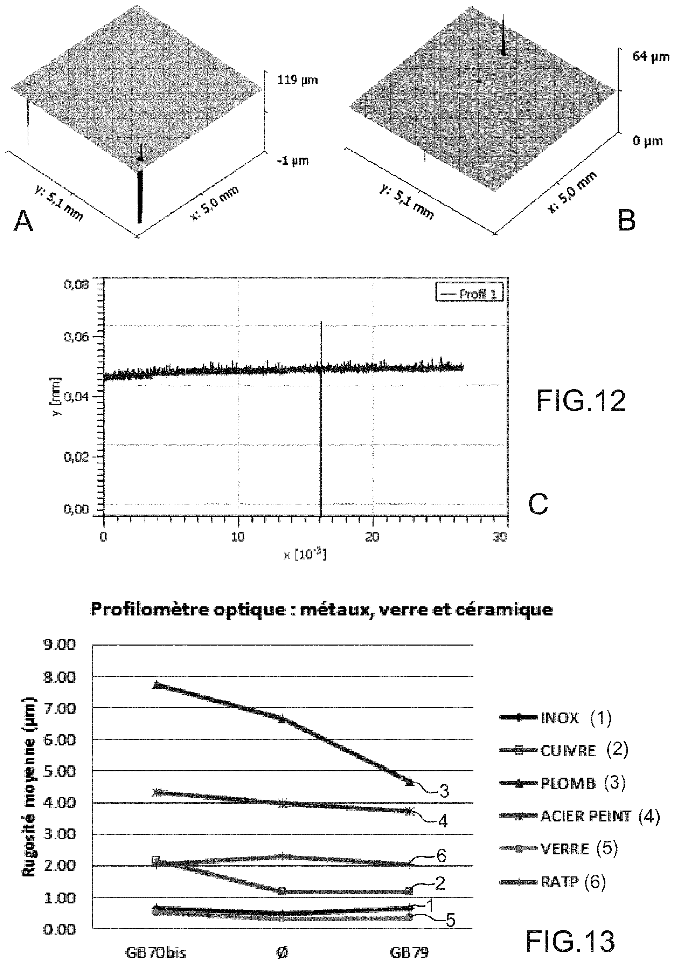

- La Figure 12 représente la cartographie 3D et le profil obtenu au profilomètre optique le long d'un support en acier inoxydable dont une partie a été traitée par le gel GB79 selon l'invention et dont une partie n'a pas été traitée par ce gel, est restée vierge.

La Figure 12A représente la cartographie 3D de la partie du support qui a été traitée par le gel selon l'invention et la Figure 12B représente la cartographie 3D de la partie du support qui n'a pas été traitée par ce gel.

Sur la Figure 12C, la partie gauche du profil avant la séparation est le profil de la partie du support traitée par le gel selon l'invention, et la partie droite du profil après la séparation est le profil de la partie du support qui n'a pas été traitée par le gel selon l'invention.
- La Figure 13 est un graphique qui donne les rugosités moyennes (en µm) mesurées au profilométre optique de la surface de supports en divers matériaux minéraux, à savoir en acier inoxydable (courbe 1 « INOX »), cuivre (courbe 2), plomb (courbe 3), acier peint (courbe 4), verre (courbe 5), et en céramique (carreau en céramique fourni par la RATP : courbe 6 « RATP »).

Chacune de ces surfaces comporte trois zones pour chacune desquelles on réalise les mesures : une première zone est traitée par le gel à l'eau inactif GB70bis, une deuxième zone n'est pas traitée (représentée par ø sur le graphique), et une troisième zone est traitée par le gel actif GB79 selon l'invention.

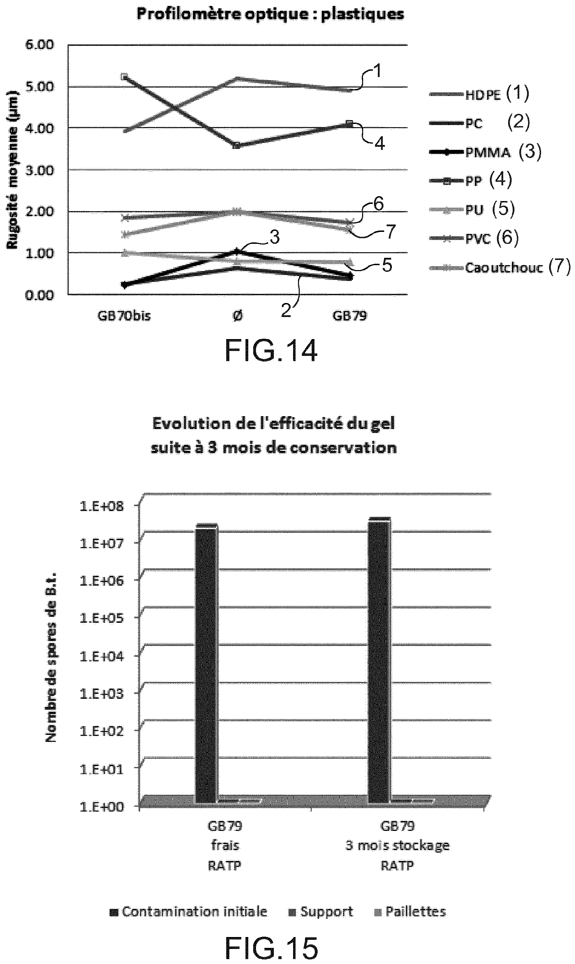

- La Figure 14 est un graphique qui donne les rugosités moyennes (en µm) mesurées au profilomètre optique de la surface de supports en divers matériaux organiques plastiques, à savoir en polyéthylène haute densité (HDPE) (courbe 1), en polycarbonate (PC) (courbe 2), en poly(méthacrylate de méthyle) (PMMA) (courbe 3), en polypropylène (PP) (courbe 4), en polyuréthane (PU) (courbe 5), en poly(chlorure de vinyle) (PVC) (courbe 6), et en caoutchouc (courbe 7).

Chacune de ces surfaces comporte trois zones pour chacune desquelles on réalise les mesures : une première zone est traitée par le gel à l'eau inactif GB70bis, une deuxième zone n'est pas traitée (représentée par ø sur le graphique), et une troisième zone est traitée par le gel actif GB79 selon l'invention.
- La Figure 15 est un graphique qui montre l'efficacité biocide d'un gel GB79 selon l'invention frais (3 barres de gauche) et après 3 mois de conservation (3 barres de droite) sur des supports qui sont des carreaux en céramique fournis par la RATP contaminés par des spores de *Bacillus thuringiensis.*

L'échelle à gauche indique le nombre de spores de *Bacillus thuringiensis* comptées.

Pour chaque gel, est porté de gauche à droite le nombre de spores déposées initialement (contamination initiale), détectées sur le support, et dans les paillettes.
- La Figure 16 est un graphique qui montre l'évolution dans le temps du pourcentage de chlore actif dans le gel GB79 selon l'invention (◆) (courbe 1), dans de l'eau de javel conservée au réfrigérateur (▲) (courbe 2), et dans de l'eau de javel conservée dans le laboratoire (x) (courbe 3).

En ordonnée est porté le % de chlore actif (% c.a.), et en abscisse est porté le nombre de jours de conservation.
- La Figure 17 est un graphique qui montre le ressuage du gel à la soude GB69 (à gauche) et du gel selon l'invention GB79 (à droite).

L'échelle à gauche indique le ressuage (en % massique).

Pour chaque gel est porté le ressuage à T0, (barre la plus à gauche) et pour des durées de stockage de 1 mois, 2 mois, et 3 mois.
- La Figure 18 montre le mode opératoire suivi pour tester l'efficacité du gel selon l'invention GB79 sur la ricine.
- La Figure 19 est un graphique (courbes de cytotoxicité) qui montre les résultats d'essais de cytotoxicité montrant l'effet de la ricine sur des cellules, et l'effet du gel GB79 selon l'invention sur la ricine. Cette cytotoxicité est évaluée en mesurant la biosynthèse de protéine par ces cellules. Plus la cytotoxicité est importante plus la biosynthèse est faible.

On a testé l'effet sur ces cellules de la ricine liquide (courbe A, en trait plein, points ●), de la ricine « séchée » après évaporation à température ambiante (courbe B, en trait pointillé, points ■), de la ricine après application sur la ricine séchée du gel GB79 selon l'invention et séchage (courbe C, en traits interrompus, points ▲), de la ricine présente dans les paillettes de gel sec (points ◆).

En ordonnée est portée la biosynthèse de protéine (en % du témoin) et en abscisse est porté le log Ricine (M).

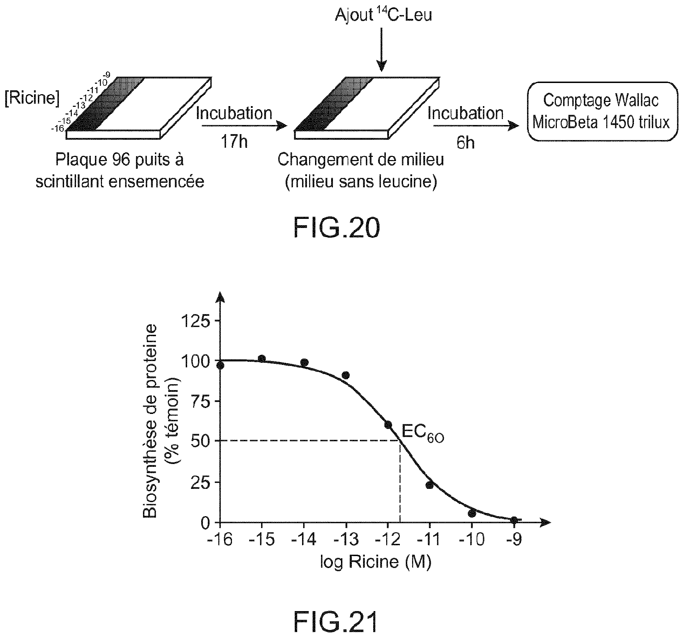

- La Figure 20 montre le principe du test de cytotoxicité décrit dans l'annexe 1.
- La Figure 21 montre un exemple de courbe de cytotoxicité.

En ordonnée est portée la biosynthèse de protéine (en % du témoin) et en abscisse est porté le log Ricine (M).

### EXPOSÉ DÉTAILLÉ DE MODES DE RÉALISATION PARTICULIERS

Le gel selon l'invention peut être facilement préparé à la température ambiante.

Par exemple, le gel selon l'invention peut être préparé en ajoutant de préférence progressivement, le ou les agent(s) viscosant(s) inorganique(s), par exemple la ou les alumine(s) et/ou la ou les silice(s), à une solution contenant l'agent actif de décontamination biologique (constitué par la combinaison d'une base inorganique et d'un agent oxydant), le ou les tensio-actif(s) éventuel(s), et le ou les pigment(s) éventuel(s). Cette solution peut être préparée par exemple en préparant tout d'abord une solution de l'agent oxydant, par exemple une solution d'hypochlorite de sodium dans de l'eau déminéralisée, puis en mélangeant à cette solution d'agent oxydant, la base minérale, le ou les tensio-actif(s) éventuels, et le ou les pigment(s) éventuel(s). Ce mélange peut être réalisé par agitation mécanique, par exemple au moyen d'un agitateur mécanique équipé d'une hélice à trois pales. La vitesse de rotation est par exemple de 200 tours/minute, et la durée de l'agitation est par exemple de 3 à 5 minutes.

L'addition du ou des agent(s) viscosant(s) inorganique(s) à la solution contenant le mélange actif de décontamination biologique, le ou les tensio-actif(s) éventuel(s), et le ou les pigment(s) éventuel(s) peut être réalisée en versant simplement le ou les agent(s) viscosant(s) dans ladite solution. Lors de l'addition du ou des agent(s) viscosant(s) inorganique(s), la solution contenant le mélange actif de décontamination biologique, le ou les tensio-actif(s) éventuel(s), et le ou les pigment(s) éventuel(s) est généralement maintenue sous agitation mécanique.

Cette agitation peut être, par exemple, réalisée au moyen d'un agitateur mécanique équipé d'une hélice à trois pales.

La vitesse d'agitation est généralement augmentée graduellement au fur et à mesure que la viscosité de la solution augmente, pour atteindre finalement une vitesse d'agitation comprise par exemple entre 400 et 600 tours/minute, sans qu'il n'y ait eu de projections.

Après la fin de l'ajout du ou des viscosant(s) minéral (aux), l'agitation est encore poursuivie, par exemple pendant 2 à 5 minutes, de manière à obtenir un gel parfaitement homogène.

Il est bien évident que d'autres protocoles de préparation des gels selon l'invention peuvent être mis en oeuvre avec une addition des composants du gel dans un ordre différent de celui mentionné plus haut.

Généralement, le gel selon l'invention doit présenter une viscosité inférieure à 200 mPa.s sous un cisaillement de 1000 s⁻¹ de manière à permettre la pulvérisation sur la surface à décontaminer, à distance (par exemple à une distance de 1 à 5 m) ou à proximité (par exemple à une distance inférieure à 1 m, de préférence de 50 à 80 cm). Le temps de reprise de la viscosité doit généralement être inférieur à une seconde et la viscosité sous faible cisaillement supérieure à 10 Pa.s pour ne pas couler sur la paroi.

Il est à noter que l'agent tensio-actif éventuel du gel selon l'invention influence favorablement et notablement les propriétés rhéologiques du gel selon l'invention. Ce tensio-actif permet notamment que le gel selon l'invention puisse être mis en oeuvre par pulvérisation et évite les risques d'épandage ou de coulure lors du traitement des surfaces verticales et des plafonds. Ce tensio-actif permet également de limiter le phénomène de ressuage observé lors de la conservation du gel.

Le gel selon l'invention ainsi préparé est ensuite appliqué (1) (Figure 1A) sur la surface solide (2) à décontaminer d'un substrat en un matériau solide (3), en d'autres termes sur la surface (2) ayant été exposée à une contamination biologique (4) ; cette contamination biologique (4) peut être constituée par une ou plusieurs des espèce(s) biologique(s) déjà définie(s) plus haut.

Hormis éventuellement les alliages de métaux légers de type aluminium, il n'existe aucune limitation quant au matériau qui constitue la surface (2) à décontaminer, en effet le gel selon l'invention permet de traiter sans aucun endommagement, toutes sortes de matériaux même fragiles.

Le gel selon l'invention ne génère aucune altération, érosion, attaque, chimique, mécanique ou physique du matériau traité. Le gel selon l'invention n'est donc en aucune manière préjudiciable à l'intégrité des matériaux traités et permet même leur réutilisation. Ainsi, des matériels sensibles tels que des équipements militaires sont préservés et pourront après leur décontamination être réutilisés, tandis que les monuments traités par le gel selon l'invention ne sont absolument pas dégradés et voient leur intégrité visuelle et structurale conservée.

Ce matériau du substrat (3) peut donc être choisi parmi par exemple les métaux ou alliages comme l'acier inoxydable, les polymères tels que les matières plastiques ou caoutchoucs parmi lesquels on peut citer les PVC, PP, PE notamment HDPE, PMMA, PVDF, PC, les verres, les ciments, mortiers et bétons, les plâtres, les briques, la pierre naturelle ou artificielle, les céramiques.

Dans tous les cas (voir Exemple 4 et Figure 7), quel que soit le matériau, l'efficacité de décontamination par le gel selon l'invention est totale.

La surface traitée peut être peinte ou non peinte.

Il n'existe également aucune limitation quant à la forme, la géométrie et la taille de la surface à décontaminer, le gel selon l'invention et le procédé le mettant en oeuvre permettent le traitement de surfaces de grande taille, de géométries complexes, présentant par exemple des creux, angles, recoins.

Le gel selon l'invention assure le traitement efficace non seulement de surfaces horizontales telles que des planchers, mais aussi de surfaces verticales telles que des murs, ou de surfaces inclinées ou en surplomb telles que des plafonds.

Par rapport aux procédés de décontamination biologiques existants qui mettent en oeuvre des liquides tels que des solutions, le procédé de décontamination selon l'invention qui met en oeuvre un gel est particulièrement avantageux pour le traitement de matériaux de grande surface, non transportables et implantés à l'extérieur. En effet, le procédé selon l'invention du fait de la mise en oeuvre d'un gel, permet la décontamination *in situ* en évitant l'épandage de solutions chimiques dans l'environnement et la dispersion des espèces contaminantes.

Le gel selon l'invention peut être appliqué sur la surface à traiter par tous les procédés d'application connus de l'homme du métier.

Des procédés classiques sont la pulvérisation par exemple au pistolet ou l'application au moyen d'un pinceau, ou d'une taloche.

Pour l'application par pulvérisation du gel selon l'invention sur la surface à traiter, la solution colloïdale peut par exemple être véhiculée par l'intermédiaire d'une pompe basse pression, par exemple une pompe qui met en oeuvre une pression inférieure ou égale à 7 bar, soit environ 7.10⁵ Pascals.

L'éclatement du jet de gel sur la surface peut être obtenu par exemple au moyen d'une buse à jet plat ou à jet rond.

La distance entre la pompe et la buse peut être quelconque, par exemple elle peut être de 1 à 50 m, notamment de 1 à 25 m.

Le temps de reprise de la viscosité suffisamment court des gels selon l'invention permet aux gels pulvérisés d'adhérer à toutes les surfaces, par exemple à des parois.

La quantité de gel déposée sur la surface à traiter est généralement de 100 à 2000 g/m², de préférence de 500 à 1500 g/m², de préférence encore de 600 à 1000 g/m².

La quantité de gel déposée par unité de surface et, par voie de conséquence, l'épaisseur du gel déposé influence la vitesse de séchage.

Ainsi, lorsque l'on pulvérise un film, couche de gel d'une épaisseur de 0,5 mm à 2 mm sur la surface à traiter, le temps de contact efficace entre le gel et les matériaux est alors équivalent à son temps de séchage, période pendant laquelle le principe actif contenu dans le gel va interagir avec la contamination.

En outre, il a été montré de manière surprenante que la quantité de gel déposée lorsqu'elle se situe dans les plages mentionnées plus haut et en particulier lorsqu'elle est supérieure à 500 g/m² et notamment dans la plage de 500 à 1500 g/m², ce qui correspond à une épaisseur minimale de gel déposée par exemple supérieure à 500 µm pour une quantité de gel déposée supérieure à 500 g/m², permettait après séchage du gel d'obtenir une fracturation du gel sous la forme de paillettes millimétriques, par exemple d'une taille de 1 à 10 mm, de préférence de 2 à 5 mm aspirables.

La quantité de gel déposée et donc l'épaisseur de gel déposé, de préférence supérieure à 500 g/m² soit 500 µm, est le paramètre fondamental qui influence la taille des résidus secs formés après séchage du gel et qui assure ainsi que des résidus secs de taille millimétrique et non des résidus pulvérulents soient formés, de tels résidus étant facilement éliminés par un procédé mécanique et de préférence par aspiration.

Cependant, il est également à noter que grâce à l'agent tensio-actif à faible concentration, le séchage du gel est amélioré et conduit à un phénomène de fracturation homogène avec une taille des résidus secs mono dispersée et une aptitude accrue des résidus secs à se détacher du support.

Le gel est ensuite maintenu sur la surface à traiter pendant toute la durée nécessaire à son séchage. Au cours de cette étape de séchage dont on peut considérer qu'elle constitue la phase active du procédé selon l'invention, le solvant contenu dans le gel, à savoir généralement l'eau contenue dans le gel s'évapore jusqu'à l'obtention d'un résidu sec et solide.

La durée de séchage dépend de la composition du gel dans les gammes de concentration de ses constituants données plus haut, mais aussi, comme on l'a déjà précisé, de la quantité de gel déposée par unité de surface c'est-à-dire de l'épaisseur de gel déposé.

La durée de séchage dépend aussi des conditions climatiques à savoir de la température, de la ventilation et de l'humidité relative de l'atmosphère dans laquelle se trouve la surface solide.

Le procédé selon l'invention peut être mis en oeuvre dans des conditions climatiques extrêmement larges, à savoir à une température T de 1°C à 50°C et à une humidité relative HR de 20% à 80%.

La durée de séchage du gel selon l'invention est donc généralement de 1 heure à 24 heures à une température T de 1°C à 50°C et à une humidité relative HR de 20% à 80%.

Il est à noter que la formulation du gel selon l'invention notamment lorsqu'elle contient des tensio-actifs tels que les « Pluronics^{®} » assure généralement un temps de séchage qui est sensiblement équivalent au temps de contact (entre l'agent de décontamination, tel qu'un agent biocide, et les espèces biologiques notamment bio-toxiques à éliminer) qui est nécessaire, requis pour inactiver et/ou absorber les espèces contaminantes polluant le matériau. En d'autres termes, la formulation du gel assure un temps de séchage qui n'est autre que le temps d'inactivation des espèces contaminantes biologiques et qui est compatible avec la cinétique d'inhibition de la contamination biologique.

La surface spécifique de la charge minérale généralement utilisée qui est généralement de 50 m²/g à 300 m²/g, de préférence de 100 m²/g et la capacité d'absorption du gel selon l'invention permettent de piéger la contamination labile (surfacique) du matériau constituant la surface à traiter.

Le cas échéant, les espèces biologiques contaminantes sont inactivées dans la phase gélifiée. Après séchage du gel, la contamination inactivée est éliminée lors de la récupération du résidu de gel sec décrite plus bas.

A l'issue du séchage du gel, le gel se fracture de manière homogène pour donner des résidus secs solides millimétriques, par exemple d'une taille de 1 à 10 mm, de préférence de 2 à 5 mm non pulvérulents, généralement sous la forme de paillettes solides (5) (Figure 1B).

Les résidus secs peuvent contenir la ou les espèce(s) contaminante(s) inactivée(s) (6).

Les résidus secs, tels que des paillettes (5), obtenus à l'issue du séchage présentent une faible adhérence à la surface (2) du matériau décontaminé. De ce fait, les résidus secs obtenus après séchage du gel peuvent être facilement récupérés par simple brossage et/ou aspiration. Toutefois, les résidus secs peuvent aussi être évacués par jet de gaz, par exemple par jet d'air comprimé.

Ainsi, aucun rinçage n'est nécessaire et le procédé selon l'invention ne génère aucun effluent secondaire.

Le procédé selon l'invention réalise donc ainsi tout d'abord une importante économie de réactifs chimiques par rapport à un procédé de décontamination par lavage avec une solution. Ensuite du fait qu'un déchet sous la forme d'un résidu sec directement aspirable est obtenu, une opération de rinçage avec de l'eau ou avec un liquide est évitée. Il en résulte bien évidemment une diminution de la quantité d'effluents produits mais aussi une simplification notable en termes de filière de traitement et d'exutoire.

En raison de la composition majoritairement minérale du gel selon l'invention et de la faible quantité de déchets produits, le déchet sec peut être stocké ou dirigé vers une filière d'évacuation sans traitement préalable.

A titre d'exemple, dans le cas courant où l'on applique 1000 grammes de gel par m² de surface traitée, la masse de déchet sec produite est inférieure à 300 grammes par m².

L'invention va maintenant être décrite en référence aux exemples suivants, donnés à titre illustratif et non limitatif.

### Exemples :

### Exemple 1 :

Dans cet exemple on décrit les gels étudiés dans les exemples 2 à 9 qui suivent.

Ces gels sont les suivants :
- Gel comparatif, non conforme à l'invention, dénommé GB70 : Il s'agit d'un gel minéral, inactif, à l'eau, comprenant de l'eau et de l'alumine.
- Gel comparatif, non conforme à l'invention, dénommé GB70 bis : il s'agit d'un gel minéral, inactif, à l'eau, comprenant de l'eau et de l'alumine comme le gel GB70, mais dont la viscosité est proche de celle des gels actifs.
- Gel comparatif, non conforme à l'invention, dénommé GB69 : il s'agit d'un gel minéral, actif, alcalin, comprenant de l'eau, de la soude 1M, de l'alumine, un tensio-actif, et de l'oxyde de fer rouge micronisée.
- Gel comparatif, non conforme à l'invention dénommé GBC01 : il s'agit d'un gel minéral, actif, alcalin, oxydant, comprenant de l'eau, de la soude 1M, de l'hypochlorite de sodium, de l'alumine, un tensio-actif, de l'oxyde de fer rouge micronisé, et un polymère super-absorbant.
- Gel conforme à l'invention dénommé GB79 : il s'agit d'un gel minéral, actif, alcalin, oxydant, comprenant de l'eau, de la soude 1M, de l'hypochlorite de sodium, de l'alumine, un tensio-actif, et de l'oxyde de fer rouge micronisé, et ne comprenant pas de polymère super-absorbant.

L'alumine est de l'alumine Aeroxide^{®} Alu C commercialisée par EVONIK INDUSTRIES d'une surface spécifique de 100 m²/g (BET), le tensio-actif est le tensio-actif Pluronic^{®} PE6200 commercialisé par BASF, la soude est de la soude 1M commercialisée par SIGMA-ALDRICH, l'hypochlorite de sodium est de l'hypochlorite de sodium à 10à 15% en chlore actif, commercialisée par SIGMA-ALDRICH, le polymère super-absorbant est le polymère super-absorbant Aquakeep^{®} produit par SUMITOMO-SEIKA, et l'oxyde de fer rouge est de l'oxyde de fer rouge micronisé disponible sous la dénomination Ferroxide^{®} 212M auprès de la société ROCKWOOD PIGMENTS LTD, de formule Fe₂O₃.

Le gel conforme à l'invention dénommé GB79 est préparé de la manière suivante : la solution d'hypochlorite de sodium est diluée à 50% avec de l'eau déminéralisée. Cette solution, le tensio-actif, l'oxyde de fer et la soude sont ensuite mélangés à l'aide d'un agitateur mécanique, muni d'un agitateur à trois pales, à une vitesse de 200 rotations/min, pendant 3 à 5 minutes. L'alumine est ensuite ajoutée progressivement dans le mélange réactionnel, en augmentant graduellement la vitesse d'agitation au fur et à mesure que la viscosité croît, pour arriver à environ 400 à 600 tours/min sans qu'il n'y ait de projections. Le gel est ensuite maintenu sous agitation pendant 5 minutes.

Les autres gels sont préparés de manière analogue.

La composition des différents gels étudiés est donnée dans le Tableau 1 ci-dessous.

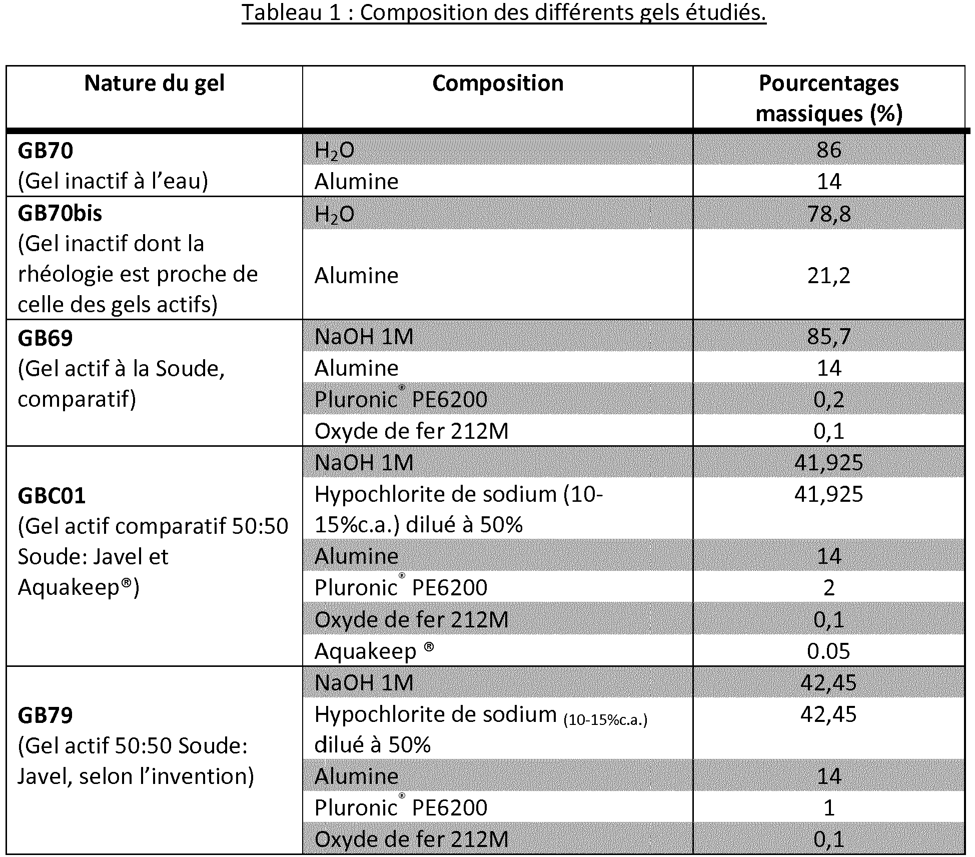

### Exemple 2 :

Dans cet exemple, on montre l'amélioration de l'efficacité biocide du gel GB79 soude-javel selon l'invention par rapport au gel comparatif GB69 qui contient seulement de la soude.

Dans cet exemple, afin de comparer l'efficacité biocide de ces deux gels, des expériences sont réalisées en laboratoire de microbiologie L2 en univers stérile -c'est à dire dans une hotte à flux laminaire- sur un *simili* de *Bacillus anthracis,* à savoir des spores de *Bacillus thuringiensis* (B.t.).

Différents supports en acier inoxydable sont nettoyés et passés à l'autoclave.

Deux d'entre eux sont salis, pollués, artificiellement afin de tenter de reproduire un matériau usagé de la façon la plus fidèle possible. Cette salissure, pollution est constituée par un mélange de 1% d'argile (Montmorillonite disponible auprès de SIGMA-ALDRICH sous la dénomination « *Aluminum Pillared Clay* »), de 10% d'huile de moteur 15W40, et d'éthanol pour le complément.

Ensuite, tous les supports sont contaminés par un dépôt liquide de 100 µL d'une solution à 2x10⁸ spores de *Bacillus thuringiensis* (B.t.) par mL, soit un dépôt de 2x10⁷ spores de B.t. que l'on laisse sécher totalement (30 minutes environ).

Le gel à tester est ensuite appliqué selon un volume calculé en fonction de la surface des supports afin d'avoir une épaisseur de gel appliquée de 0,7mm. Les supports sont ainsi mis à sécher dans des boîtes de Pétri closes jusqu'au séchage complet du gel (3-5 heures en fonction de la température du laboratoire).

Ensuite, les paillettes sont récupérées dans un tube Falcon par brossage dans une quantité connue de milieu nutritif Luria-Broth (LB). De même, les supports sont placés dans un volume connu de LB dans un tube Falcon. L'ensemble des tubes Falcon sont ensuite vortexés, puis placés dans un incubateur durant 1h à 30°C sous agitation.

Ensuite, les tubes Falcon contenant des paillettes sont centrifugés (3 min, 4500 rpm).

Puis, pour chacun des tubes, une gamme de dilutions au dixième est réalisée à partir du surnageant. Enfin, on prélève 1mL dans chacun des tubes de chaque gamme de dilutions. Le prélèvement est ensuite déposé au fond d'une boîte de Pétri vide et stérile. Du milieu LB gélosé est ensuite coulé dans la boîte (ensemencement dans la masse). On place ensuite ces boîtes dans l'incubateur à 30°C pour 24 heures. Les colonies dans les boîtes sont ensuite comptées une à une, puis pour chaque échantillon (support ou paillettes à partir duquel la gamme de dilutions a été réalisée), une moyenne de spores vivantes est calculée. Enfin, les différentes dilutions sont prises en compte pour obtenir le nombre de spores vivantes total présentes sur le support ou dans les paillettes. Le facteur de décontamination peut ensuite être calculé en déterminant l'abattement en millier de spores tuées (log₁₀).

Dans cet exemple, le but étant de comparer l'efficacité biocide du gel biocide GB79 selon l'invention au gel comparatif GB69, les gels GB70bis (gel inactif à l'eau), GB69 (gel comparatif) et GB79 (gel selon l'invention) sont testés selon le protocole décrit ci-dessus.

Les résultats de ces expériences sont représentés sur la Figure 3 où apparaît le facteur de décontamination obtenu sur les supports en acier inoxydable en fonction du gel utilisé (voir aussi Figures 2A, 2B, 2C, 2D).

Sur cet histogramme, il apparaît que le gel comparatif GB69 a la même efficacité biocide que le gel sans agent actif de décontamination, à savoir le gel à l'eau GB70bis. En revanche, le gel selon l'invention GB79, possède, aussi bien sur support propre que sur support pollué, une efficacité biocide de 7 log minimum. En effet, aucune spore vivante résiduelle n'a été détectée lors des comptages sur les 2x10⁷ spores initialement déposées. La décontamination de la surface à traiter à l'aide du gel selon l'invention, dont l'activité biocide est renforcée est donc efficace même sur un support pollué, montrant ainsi son fort pouvoir dégraissant.

### Exemple 3 :

Dans cet exemple, on montre l'incompatibilité entre l'agent oxydant et le polymère super-absorbant.

Dans la formulation de gel biocide du document [1] du polyacrylate de sodium, qui est polymère super-absorbant, avait été ajouté afin d'améliorer l'efficacité du gel biocide sur matériaux poreux tels que les mortiers. En effet, cet adjuvant permet une libération prolongée de l'actif de décontamination. Cependant, la rhéologie de ce type de gel est fortement modifiée au point de devenir très compacte. Le contact devient alors très mauvais sur les surfaces à décontaminer.

Dans cet exemple, on compare tout d'abord l'efficacité biocide de deux gels contenant de la javel et de la soude. Le premier gel est un gel conforme à l'invention, formulé sans polymère super-absorbant (GB79), le second est un gel comparatif qui contient un polymère absorbant (GBC01), et qui a été conservé plus de 30 jours.

L'efficacité biocide est évaluée exactement selon le même protocole que dans l'exemple 2, sauf que la contamination initiale en spores déposées est de 2x10⁷ pour les supports traités au gel GB79, et de 7,5x10⁶ pour les supports traités au gel GBC01.

Les supports traités par les gels sont des supports en acier inoxydable (appelés supports INOX) et des supports constitués par des carreaux en céramique du type de ceux qui revêtent les parois des stations de métro parisien et qui sont fournis par la RATP (appelés supports RATP).

Les résultats, représentés sur la Figure 4, montrent une baisse de l'efficacité biocide pour le gel contenant le polymère super-absorbant (PSA), pour lesquels, pourtant, les supports étaient légèrement moins contaminés.

En effet, avec le gel sans PSA selon l'invention (GB79), les supports INOX et RATP sont décontaminés d'au moins 7 log, soit la quantité initiale déposée.

En revanche, avec le gel contenant un PSA (GBC01), la décontamination des supports atteint difficilement 5 log, sachant que la contamination initiale était moins élevée.

A noter également que dans tous les cas, les paillettes ne contiennent aucune spore vivante détectable.

On étudie ensuite la rhéologie du gel conforme à l'invention, formulé sans polymère super-absorbant (GB79), et du gel comparatif qui contient un polymère absorbant (GBC01).

Plus particulièrement, on mesure la contrainte seuil et la viscosité des gels GB69, GB79, GBC01 frais (venant d'être préparé) aussi appelé gel neuf, et GBC01 conservé plus d'un mois, aussi appelé gel ancien.

La mesure de la viscosité en fonction du taux de cisaillement est réalisée à l'aide d'un viscosimètre Rheomat^{®} RM100 de la société LAMY RHEOLOGY. Le viscosimètre est équipé d'un système de mesure de type ancre MS-R3. Après un pré-cisaillement de 10 secondes à un taux de cisaillement de 1s⁻¹, 15 paliers de taux de cisaillement allant de 1s⁻¹ à 100s⁻¹ sont effectués avec mesure de la viscosité toutes les 20 secondes.

La mesure de la contrainte seuil est réalisée à l'aide d'un rhéomètre TA Instruments AR-1000 en géométrie « Vane ». Un faible taux de cisaillement (6.7x10⁻³ S⁻¹) est appliqué aux gels de manière constante afin de les déformer en partant du repos et ainsi de déterminer leur seuil d'écoulement.

Les résultats sont représentés sur les Figures 5 et 6.

Il apparaît sur la Figure 5, qui représente la viscosité en fonction du taux de cisaillement en échelle logarithmique, que les deux courbes des gels GB69, et GB79 selon l'invention, sans polymère super-absorbant sont très proches et parallèles. Elles sont d'ailleurs linéaires ce qui correspond au comportement rhéologique des fluides rhéofluidifiants avec contrainte seuil.

En revanche, pour les deux gels GBC01 frais et conservé qui contiennent un PSA, les courbes ne sont pas linéaires (cf. coefficients de régression) ce qui caractérise un comportement rhéologique moins idéal et prévisible que celui du gel selon l'invention sans polymère super-absorbant.

La Figure 6 représente la contrainte de cisaillement en fonction de la déformation pour chacun des gels. Dans tous les cas, deux régimes sont notables. Tout d'abord la contrainte augmente linéairement, le matériau est en régime solide (déformation élastique). On observe ensuite un changement de comportement, la contrainte atteint le seuil d'écoulement et le matériau passe en régime liquide (écoulement stationnaire). La contrainte seuil correspond à la contrainte au seuil d'écoulement, soit 106,5 Pa pour le gel GBC01 neuf (courbe **1**), 49,35 Pa pour le gel GBC01 conservé (courbe **2**), mais dont l'allure n'est pas conforme aux profils classiques et dont la valeur est fortement discutable, 49,69 Pa pour le gel GB69 (courbe **3**) et 39,13 Pa pour le gel GB79 (courbe **4**).

Cet exemple permet donc de montrer qu'avec le gel biocide selon l'invention, il est possible de s'affranchir de la présence d'un polymère superabsorbant, tel que le polyacrylate de sodium, car il n'améliore pas visiblement l'efficacité du gel biocide tout en altérant sa rhéologie, en effet le gel qui contient un PSA est un gel très visqueux dont le comportement rhéofluidifiant est peu prévisible, notamment suite à plusieurs jours de conservation après lesquels la mesure de contrainte seuil devient impossible.

### Exemple 4 :

Dans cet exemple, on montre l'efficacité biocide du gel GB79 selon l'invention sur différents supports, en divers matériaux.

Dans cet exemple, l'efficacité biocide est évaluée selon le protocole de l'exemple 2, à la différence que la contamination initiale est de 2x10⁷ spores de B.t. déposées sur tous les supports, sauf sur les deux supports en plastique où elle est de 2x10⁶.

De plus, tous les supports sont propres. Les différents supports testés sont les suivants : un support en verre (appelé support VERRE), un support en acier inoxydable, un support constitué par un carreau en céramique fourni par la RATP, un support en mortier, un support en PVC (polychlorure de vinyle), et un support en PVDF (polyfluorure de vinylidène).

Les résultats sont présentés sur la Figure 7. Ils montrent que sur les supports en matériaux non poreux (supports VERRE, INOX et RATP), la décontamination des supports atteint 6-7 log au minimum.

Sur les supports en mortier et les supports en matières plastiques, près de 5 log de spores sont tuées (rappelons que dans l'exemple 2, sur des supports en acier inoxydable, la décontamination n'atteignait pas 2 log avec le gel GB69 à la soude). Concernant les résidus secs, c'est-à-dire les paillettes, dans tous les cas, aucune spore résiduelle n'est détectable.

### Exemple 5 :

Dans cet exemple, on montre l'absence de spores vivantes dans les résidus secs, les paillettes.

Plus exactement, dans cet exemple, on montre qu'on ne trouve effectivement aucune spore résiduelle dans les paillettes, c'est-à-dire qu'aucune spore vivante ne se trouve emprisonnée dans les paillettes sans pouvoir apparaître au comptage car elles seraient piégées dans ces dernières et ne migreraient pas dans le milieu LB.

Pour ce faire, sur deux supports RATP propres, et toujours selon le protocole détaillé dans l'exemple 2 (excepté la contamination initiale qui est ici constituée par un dépôt de 10⁶ spores de B.t.), on effectue un séchage du gel selon l'invention GB79.

A la fin du séchage, sur le premier support les paillettes sont récupérées classiquement par brossage dans une quantité connue de milieu LB.

Sur le second support, les paillettes sont brossées puis broyées finement au mortier avant d'être mises en contact avec du milieu LB. La suite du protocole est ensuite classique, à savoir : incubation 1h à 30°C, « vortex », centrifugation, gammes de dilutions, boîtes de comptage, incubation 24h à 30°C (cf. protocole de l'exemple 2).

Les résultats des comptages sur ces deux séries de paillettes sont représentés sur la Figure 8 et sont confrontés aux résultats des manipulations précédentes sur différents matériaux propres. Quelque-soit le matériau, aucune spore vivante n'est décelable dans les paillettes. Cela est également confirmé de nouveau lorsque les paillettes sont finement broyées (dernière barre de l'histogramme).

### Exemple 6 :

Dans cet exemple, on montre la cinétique d'action du gel selon l'invention GB79. Pour ce faire, différentes manipulations sont réalisées sur 10 supports en céramique RATP propres.

La contamination initiale des supports est de 10⁷ spores de B.t. par support.

La même manipulation est réalisée avec un gel à l'eau GB70bis.

Les gels sont appliqués sur les différents supports au temps T0 = 0 minute.

Ensuite, les gels en phase de séchage, voire en phase de fracturation, sont récupérés au bout de 0min, 10min, 20min, 30min, et 1h.

Lors de chaque opération de récupération, le gel et le support sont récupérés dans des quantités connues de milieu de culture LB avant de suivre le traitement classique, à savoir : incubation, vortex, centrifugation, gamme de dilutions, boites de comptage, incubation 24h à 30°C (cf. protocole de l'exemple 2).

Les résultats sont présentés sur la Figure 9. Il apparaît que le gel actif GB79 selon l'invention décontamine le support de plus de 3 log dans les 10 premières minutes, et que, au temps de séchage complet de 210 minutes, ce gel décontamine avec une efficacité d'au moins 7 log.

Ces résultats peuvent être comparés avec les résultats obtenus lors de la même expérience réalisée avec un gel à l'eau inactif dans laquelle aucune décontamination n'est notable au cours du temps.

Concernant les paillettes du gel GB79, il apparaît qu'au bout de 10 minutes, aucune spore résiduelle n'est détectable. Cela confirme une nouvelle fois les résultats obtenus dans l'exemple 5. En effet, de 0 à 60 minutes, le gel n'est pas encore fracturé et est humide. Il est donc aisé de le remettre en solution de manière homogène dans le milieu nutritif LB lors de sa récupération pour compter le nombre de spores vivantes. Les résultats montrent une fois encore, alors que le gel est totalement en solution dans le LB et qu'aucune spore n'y est détectée, qu'aucune spore vivante résiduelle ne peut échapper à la détection du fait de son emprisonnement dans le réseau solide que forment les paillettes (qui se dissolvent très mal une fois le séchage total complètement atteint), et que les paillettes récupérées ne sont donc pas contaminées.

### Exemple 7 :

Dans cet exemple, on montre que le gel GB79 selon l'invention est particulièrement bien adapté à une mise en oeuvre par pulvérisation.

L'étude rhéologique du gel GB79 permet de mesurer sa contrainte seuil qui est de 39,13 Pa (cf. exemple 3, Figure 6).

Ainsi, il apparaît que les gels selon l'invention dont le principe actif est un mélange de Javel et de Soude remplissent le cahier des charges des « gels aspirables », soit une contrainte seuil supérieure à 15-20 Pa pour que le gel ne s'écoule pas sous l'effet de la gravité sur une paroi verticale pour des épaisseurs de 0,5-2mm.

Par ailleurs, la viscosité du gel selon l'invention (cf. exemple 3, Figure 5) est totalement similaire et très proche de celle du gel à la soude GB69 qui a déjà été testé par une mise en oeuvre par pulvérisation. Le gel selon l'invention est donc conforme au cahier des charges d'un « gel aspirable » d'un point de vue rhéologique.

### Exemple 8 :

Dans cet exemple, on montre que le gel selon l'invention peut effectivement être défini, en termes de cinétique de séchage et de fracturation, comme un « gel aspirable », c'est-à-dire qu'il sèche dans un temps raisonnable, par exemple de quelques heures, et qu'il se fracture en produisant des paillettes non pulvérulentes.

Ces deux caractéristiques des « gels aspirables », et plus particulièrement la cinétique de séchage, sont étroitement liées aux conditions climatiques de l'environnement de séchage, à savoir la température, l'humidité relative, et la ventilation/aération.

Dans cet exemple, les deux gels GB69 (gel à la soude) et GB79 selon l'invention (gel à la soude et à la javel) sont mis à sécher l'un après l'autre dans une enceinte climatique Binder^{®} réglée à 25°C et à 50% d'humidité relative.

Les gels sont étalés sur des nacelles en acier inoxydable usinées de façon à obtenir une épaisseur contrôlée de 0,5mm de gel dans la nacelle.

Dans l'enceinte climatique, une balance de précision Sartorius^{®} est installée, de même qu'une caméra Moticam^{®} entourée d'une lampe à DEL circulaire (VWR^{®}) qui est placée sur le dessus de la balance. La balance et la caméra Moticam^{®} sont reliées à un ordinateur placé en dehors de l'enceinte climatique, ce qui permet ainsi l'acquisition simultanée, au cours du séchage en atmosphère contrôlée, de la masse et des images de la nacelle remplie de gel.

Il est à noter que la nacelle contenant le gel est placée dans la balance de précision, et que toutes les portes de la balance sont fermées, exception faite de la porte opposée à la soufflerie, qui est ouverte de 3 cm pour maintenir une atmosphère contrôlée dans l'enceinte de la balance tout en limitant le flux d'air lié au fonctionnement de l'enceinte climatique.

L'enregistrement de la masse au cours du séchage permet alors de tracer une courbe représentant la cinétique de séchage, alors que l'analyse des images à l'aide d'un logiciel de traitement d'image du gel totalement sec permet de détecter automatiquement les paillettes et de les comptabiliser tout en calculant leur aire.

Les résultats sont présentés sur les Figures 10 et 11.

Les résultats, présentés sur la Figure 10, montrent des courbes de perte de masse complètement parallèles entre les deux gels qui atteignent un séchage total en moins de 5 heures (300 min) dans ces conditions de température et d'humidité relative. En effet en 260 à 300 minutes, les gels GB69, et GB79 selon l'invention, perdent respectivement 78% et 73% de leur masse initiale. L'ajout d'hypochlorite de sodium à la formulation n'impacte donc pas le temps de séchage total du gel qui reste largement applicable selon le procédé « gel aspirable » avec le gel selon l'invention GB79.

Concernant la fracturation, dont les résultats sont résumés sur la Figure 11, il apparaît que le nombre de paillettes est moins important pour les paillettes issues du gel GB79 selon l'invention contenant de l'hypochlorite de sodium. Les paillettes sont pour ce gel GB79 selon l'invention, en moyenne plus grandes, mais restent millimétriques (4 mm² en moyenne). Ce gel reste donc adéquat pour l'application visée puisqu'il produit des paillettes de taille millimétrique non pulvérulentes.

### Exemple 9 :

Dans cet exemple, on montre l'innocuité du gel selon l'invention GB79 sur différents matériaux.

Plus exactement, dans cet exemple, on montre que le gel selon l'invention peut être appliqué sur de nombreux matériaux sans en altérer ni les propriétés mécaniques, ni l'intégrité physique.

Pour ce faire, l'état de surface, et notamment la rugosité, de différents matériaux, est comparé pour des surfaces non traitées, des surfaces sur lesquelles le gel à l'eau inactif GB70bis a séché, ou encore des surfaces traitées par le gel alcalin oxydant selon l'invention GB79.

Un profilomètre STIL (Sciences et Techniques Industrielles de la Lumière) est utilisé pour tracer des profils et mesurer la rugosité moyenne sur les surfaces de pièces, supports en ces différents matériaux.

La surface de chaque matériau testé se voit divisée en trois parties : la première sur laquelle sèche le gel à l'eau GB70bis, la seconde où rien n'est appliqué et la troisième sur laquelle sèche le gel GB79. Une fois les gels totalement secs, les supports sont débarrassés des paillettes et bien nettoyés avant de faire les mesures au profilomètre. Les matériaux testés sont les suivants : acier inoxydable, cuivre, plomb, acier peint, verre, céramique, RATP, HDPE (polyéthylène haute densité), PC (polycarbonate), PMMA (polyméthacrylate de méthyl), PP (polypropylène), PU (polyuréthane), PVC (polychlorure de vinyle), PVDF (polyfluorure de vinylidène) et caoutchouc.

Les résultats sont présentés sur les Figures 12 (A, B, C), 13, et 14.

La Figure 12 représente la cartographie 3D et le profil obtenu au profilomètre optique d'un support en acier inoxydable. On constate qu'il n'y a aucune modification de la rugosité (du profil) entre la partie qui a été traitée par le gel alcalin oxydant (cartographie 3D de gauche (Figure 12A)) et la partie gauche du profil avant la séparation (Figure 12C) et la partie non traitée, restée vierge (cartographie 3D de droite (Figure 12B)) et la partie droite du profil après la séparation (Figure 12C).

Les Figures 13 et 14 représentent de manière condensée les résultats de ces mesures au profilomètre optique sur l'ensemble des matériaux. Pour obtenir ces courbes, la rugosité moyenne a été mesurée sur une partie de l'échantillon dont la surface comporte trois zones, la première traitée par le gel inactif à l'eau, la seconde non traitée, et la dernière traitée par le gel selon l'invention. Pour l'ensemble des matériaux, aucune altération de la surface n'est observable à l'oeil nu. La rugosité mesurée reste relativement constante pour les différents matériaux sur les surfaces traitées et non traitées.

### Exemple 10 :

Dans cet exemple, on évalue la conservation de l'activité biocide après le stockage du gel selon l'invention GB79.

Pour évaluer la conservation de l'activité biocide suite au stockage du gel GB79, deux expériences différentes sont réalisées.

La première expérience consiste à réévaluer l'efficacité biocide du gel GB79 sur des spores de *Bacillus thuringiensis* selon le protocole exposé dans l'exemple 2 après 3 mois de stockage, conservation, à la température ambiante, sans que le gel ne soit protégé de la lumière, et à comparer les résultats ainsi obtenus, à ceux obtenus 3 mois plus tôt avec le même gel fraîchement fabriqué. Cette manipulation a été réalisée sur des supports constitués par des carreaux en céramique fournis par la RATP.

La seconde consiste à mesurer le pourcentage de chlore actif présent dans le gel afin d'évaluer sa vitesse de dégradation au cours du stockage. Pour cela, un gel frais a été fabriqué puis stocké à l'abri de la lumière dans le laboratoire. De la même manière, la solution d'hypochlorite de sodium commerciale utilisée (10-15% c.a.) est stockée au réfrigérateur et au laboratoire. De manière régulière, une petite quantité de ce gel et de cette solution sont récupérées et dissoutes dans de l'eau distillée. L'hypochlorite de sodium est ensuite dosé classiquement par un dosage en retour du diode (formé par l'addition d'iodure de potassium) par le thiosulfate de sodium.

Les résultats issus du test sur des spores de B.t. sont présentés sur la Figure 15. Après 3 mois de conservation, le gel reste aussi actif que le gel fraichement préparé.

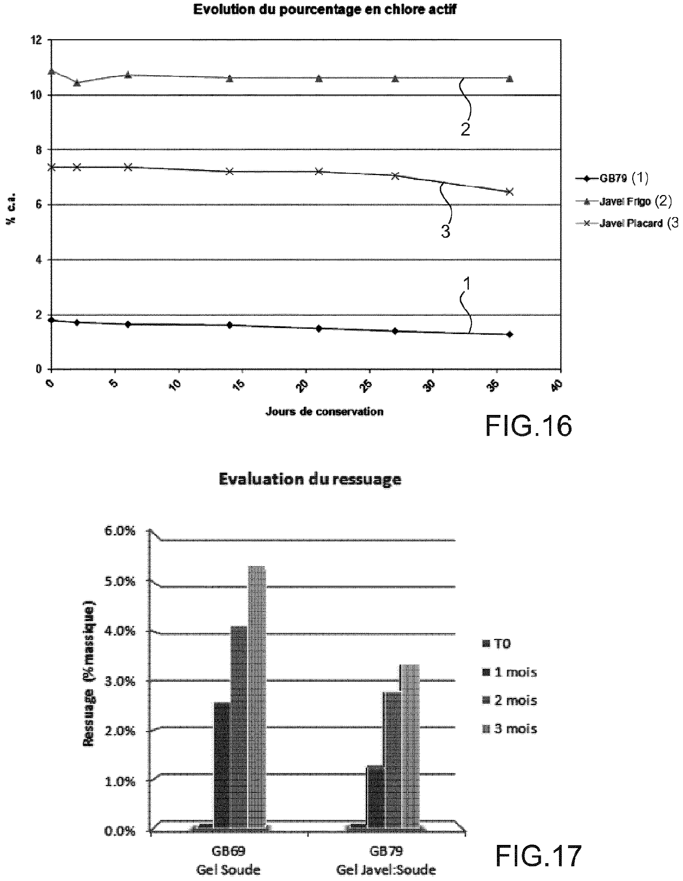

Concernant le dosage de l'hypochlorite de sodium dans le gel, dont les résultats sont présentés sur la Figure 16, il apparaît que sur plus d'un mois de conservation, le pourcentage de chlore actif (c.a.) est légèrement affecté. En effet, on observe une légère baisse du pourcentage en chlore actif dans le gel qu'il convient de surveiller pour s'assurer qu'il ne soit pas significatif. Néanmoins, au vu des résultats obtenus en testant l'efficacité biocide sur B.t. après 3 mois de conservation du gel, cette légère décroissance dans le pourcentage de chlore actif dans le gel ne semble pas affecter l'efficacité biocide de la formulation renforcée.

Cet exemple montre que le gel selon l'invention peut être stocké après sa préparation en vue d'une mise en oeuvre ultérieure en cas de besoin.

### Exemple 11 :

Dans cet exemple, on évalue le ressuage du gel selon l'invention.

En effet, un autre phénomène qu'il est important de considérer en cas de conservation du gel en vue d'une utilisation ultérieure est son ressuage, c'est-à-dire la sédimentation occasionnée par un long stockage et qui nécessite éventuellement que l'on homogénéise de nouveau le produit avant son utilisation.

Pour évaluer ce phénomène, 90 g du gel GB79 selon l'invention sont stockés sans être utilisés ou homogénéisés. La quantité de surnageant est mesurée régulièrement au cours du temps afin de quantifier ce phénomène. De la même façon, cette mesure est réalisée pour le gel GB69 afin de constater l'impact éventuel sur le ressuage de l'addition d'hypochlorite de sodium à la formulation.

Les résultats sont présentés sur la Figure 17. Il apparaît que, certes le gel selon l'invention GB79 est également victime de ce phénomène à hauteur de 3,3% en trois mois, mais que ce phénomène est visiblement plus important sur le gel GB69 qui atteint un ressuage de plus de 5% en trois mois.

Les gels Javel/Soude selon l'invention ont donc une aptitude au stockage analogue, voire supérieure, aux gels de l'ancienne formulation.

### Conclusion des exemples 1 à 11 :

Au vu des exemples présentés plus haut, il apparaît que le gel selon l'invention, du fait notamment de l'addition d'hypochlorite de sodium, est un produit efficace aussi bien en ce qui concerne sa formulation que pour sa mise en oeuvre dans le cadre de la décontamination biologique.

En effet, l'activité biocide du gel selon l'invention est renforcée par rapport à un gel ne contenant que de la soude en tant qu'agent de décontamination biologique puisqu'il permet d'atteindre des facteurs de décontamination sur des *simili* de spores d'anthrax d'au moins 6 log tout en évitant l'addition de polymère super-absorbant qui rendait impropre le gel du document [1] à une utilisation par pulvérisation après stockage.

Par ailleurs, le gel selon l'invention peut être stocké puis utilisé selon le concept d'emploi dit « gel aspirable » puisque la viscosité et sa contrainte seuil restent adaptées à une application par pulvérisation sur des parois horizontales ou verticales et que le gel sèche et se fracture en paillettes millimétriques non pulvérulentes dans un temps raisonnable et adapté à une intervention de type post-évènementielle suite à une attaque biologique malveillante.

Dans les exemples 12 à 15 qui suivent, le gel GB79 selon l'invention est testé sur des agents biologiques pathogènes réels afin de montrer son efficacité sur de véritables agents de la menace NRBC.

Dans ce but, des essais visant à montrer l'efficacité du gel GB76 selon l'invention ont été réalisés sur des supports contaminés par de la ricine (toxine), des spores de Bacillus anthracis (B.a.) (anthrax, maladie du charbon), des bactéries Yersinia pestis (Y.p.) (peste) et le virus de la vaccine.

### Exemple 12 :

Dans cet exemple, on montre l'efficacité décontaminante du gel biocide GB79 selon l'invention sur un agent biologique pathogène de type phytotoxine, la ricine. Cette toxine, sous-produit du traitement des graines de ricin, inhibe les cellules chargées de la synthèse de protéines dans l'organisme, pouvant ainsi entrainer la mort.

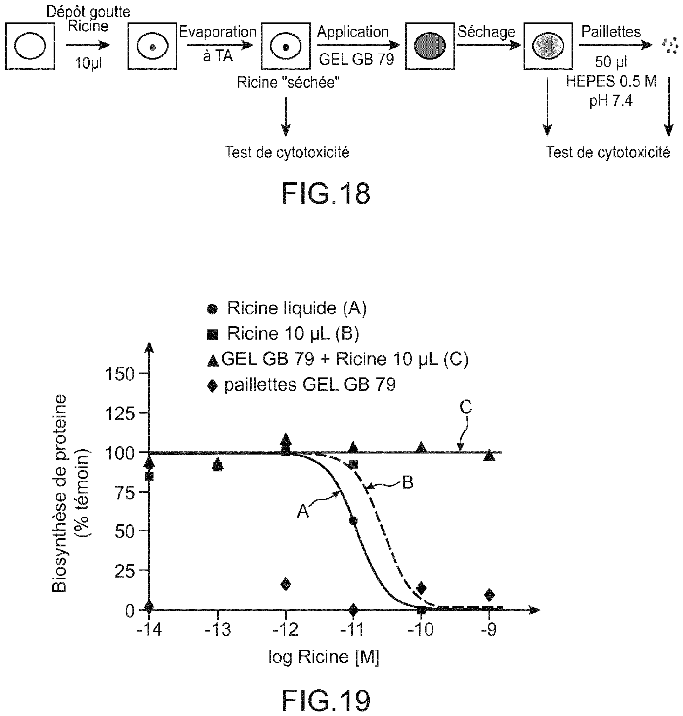

L'efficacité du gel sur la ricine a été testée sur des lames de verre contaminées par 10µL de différentes solutions de ricine (plus ou moins concentrées) (voir Figure 18).

Des essais de cytotoxicité (cf. protocole ci-après) ont été menés sur des cellules Vero afin de détecter l'activité de la ricine sans et avec application du gel biocide (la ricine empêche les cellules de fabriquer plus ou moins d'une protéine).

Les résultats de ces essais sont présentés sur la Figure 19.

La courbe A, en trait plein, points ●montre l'effet de la ricine liquide à diverses concentrations sur ces cellules.

La courbe B, en trait pointillé, points ■ montre l'effet de la ricine séchée (comme sur les lames de test) à diverses concentrations sur ces cellules.

La courbe C, en traits interrompus, points ▲, montre l'effet de la ricine suite à l'utilisation du gel GB79 sur la ricine.

On observe que le gel est capable d'inactiver efficacement la ricine sur du verre (au moins d'un facteur 1000 : pas de létalité (et donc de perte de synthèse de protéine) quand le gel est appliqué sur la ricine).

Enfin, les points ◆ représentent les paillettes mais malheureusement la toxicité résiduelle des paillettes seules (sans ricine) a un effet létal important sur les cellules et ces résultats ne sont donc pas représentatifs de la ricine éventuellement active dans les paillettes.

### Exemple 13 :

Dans cet exemple, on teste l'efficacité du gel biocide GB79 selon l'invention sur des supports contaminés par des spores de *Bacillus anthracis (B.a),* des bactéries *Yersinia pestis (Y.p)* ou encore le virus de la vaccine. Les essais sont réalisés dans des conditions de référence.

Les supports utilisés sont des coupons propres en acier inoxydable (INOX) et des carreaux en céramique (RATP) de 5cmx5cm.

Les essais réalisés dans les conditions de référence sont les tests réalisés sur les matériaux propres à la température ambiante à savoir autour de 20°C, et à 40% d'humidité relative.

Les essais sont réalisés selon le protocole suivant :
1) contamination des supports (qui sont disposés à l'horizontale dans des boîtes de Pétri tout au long de l'essai) par dépôt à la micropipette de 100 µL de suspension bactérienne ou virale sous la forme de gouttelettes ;
2) séchage de la contamination ;
3) dépôt à l'aide d'une pipette d'environ 2 à 3 mL de gel GB79 selon l'invention sur les coupons, puis étalement de celui-ci à l'aide d'étaleurs en plastique stériles ;
4) séchage du gel à la température préconisée pour l'essai. Cette étape doit être maintenue jusqu'à séchage complet du gel ;
5) récupération des particules de gel séché dans une boîte de Pétri ;
6) écouvillonnage de la totalité de la surface des supports à l'aide d'un écouvillon humidifié ;
7) extraction des écouvillons dans 2 mL d'eau stérile pour les bactéries, ou de milieu de culture pour les virus, par agitation au vortex;
8) « essuyage » des écouvillons extraits sur milieu de culture gélosé (étape non réalisée pour les virus, car la vaccine ne peut être cultivée sur un milieu « solide ») ;
9) réalisation d'une « empreinte » des supports écouvillonnés à l'aide de géloses-contacts (non réalisé pour les virus) ;
10) remise en suspension de l'ensemble des particules de gel séché dans 4 mL d'eau pour les bactéries, ou 2 mL de milieu de culture pour les virus, puis extraction par agitation au vortex ;
11) dénombrement des micro-organismes contenus dans chacune des suspensions récupérées au cours de l'essai, par mise en culture sur/dans un milieu adapté à l'agent biologique testé.

Pour chaque essai, 5 supports -coupons ou carreaux- d'essais sont réalisés, ainsi que 3 supports -coupons ou carreaux- témoins. Les supports témoins subissent les mêmes étapes que les supports d'essais, excepté la décontamination par le gel et toutes les étapes qui y sont liées.

Ainsi, seules les étapes n° 1, 2, 6, 7 et 11 leurs sont appliquées. Les supports sont également soumis aux mêmes conditions que les supports d'essais durant le séchage du gel (notamment par exemple les conditions de température, d'hygrométrie et de temps d'attente).

Les résultats sont présentés dans le Tableau 2. Il apparaît que quelque soit le pathogène, les supports sont parfaitement décontaminés, puisqu'on est en-dessous de la limite de détection des micro-organismes. Concernant les paillettes, elles ne sont pas contaminées dans l'ensemble des cas, exception faite d'un cas, où les paillettes contiennent très peu de spores d'anthrax (comparativement aux 2,4.10⁶ spores déposées initialement). A noter que dans ces conditions ambiantes de température et d'humidité relative, le gel met entre 4h et 6h pour sécher complètement sous PSM (Poste de Sécurité Microbiologique) dans des boîtes de Pétri ouvertes.

**Tableau 2 : Efficacité du gel biocide selon l'invention sur des agents pathogènes (conditions de référence).**

| Agent | Support | Température (°C) | Contamination initiale du support (CFU/PFU)(1) | Contamination finale du support (CFU/PFU) | Contamination résiduelle des paillettes (CFU/PFU) |
|---|---|---|---|---|---|
| *B*. *anthracis* (spores) | Acier inoxydable | ∼20 | 7,4.10⁶ | < I.d. (2) | < I.d. |
| | Céramique RATP | ∼20 | 2,4.10⁶ | < I.d. | 68 |
| *Y. pestis* (bactéries) | Acier inoxydable | ∼20 | 1,2.10⁶ | < I.d. | < I.d. |
| | Céramique RATP | ∼20 | 4,1.10⁶ | < I.d. | < I.d. |
| Vaccine (virus) | Acier inoxydable | ∼20 | 8,8.10⁴ | < I.d. | < I.d. |
| | Céramique RATP | ∼20 | 4,4.10⁴ | < I.d. | < I.d. |

| | | | | | |
|---|---|---|---|---|---|
| (1) CFU = Colony-forming unit, PFU = Plaque-forming unit. (2) I.d. = limite de détection (1 CFU pour spores et bactéries sur supports, 60 CFU pour spores et bactéries dans les paillettes, 20 PFU pour les particules virales sur supports et 10 PFU pour les particules virales dans les paillettes). | | | | | |

### Exemple 14 :

Dans cet exemple, on évalue l'efficacité biocide du gel selon l'invention sur les deux souches bactériennes *B*.a. et *Y.p.* dans des conditions extrêmes de température, soit 5°C et 50°C sur les mêmes matériaux que dans l'exemple 13. Le protocole est le même que celui de l'exemple précédent exception faite des conditions de séchage du gel qui sont les suivantes :
- pour les essais à 5°C, le séchage du gel est effectué en chambre froide durant 24h (les supports sont placés dans des récipients fermés pour éviter de contaminer la chambre froide). Puis les boîtes sont placées sous PSM à température ambiante pour terminer le séchage (car à 5°C le gel est infiniment long à sécher sans ventilation, dans une enceinte fermée).
- pour les essais à 50°C, les coupons dans leur boîte de Pétri sont placés dans une étuve durant le séchage du gel. Ces boîtes ont été légèrement entrouvertes.

A noter que le séchage de la contamination sur les supports, préalablement à l'application du gel, se fait sous PSM à température ambiante.

Les résultats sont présentés dans le Tableau 3.

Concernant les conditions de séchage à 5°C, on constate que côté supports et paillettes, aucune contamination résiduelle n'est détectable. Le séchage prolongé du gel, lié aux conditions basses de température, renforce la puissance de décontamination du gel. Concernant les conditions de séchage à 50°C, le gel met environ 3h30 à sécher totalement. A cette température, côté supports, la décontamination est totale sur quasiment l'ensemble des coupons, exception faite d'une très légère contamination résiduelle sur un support de céramique. Côté résidus solides, de légères contaminations résiduelles sont décelables dans certains cas. Quoiqu'il en soit, concernant les bactéries, supports et paillettes sont totalement sains. Pour les spores, micro-organismes beaucoup plus résistants, la décontamination des supports est pleinement satisfaisante, soit par annihilation des spores, soit par leur transfert dans la phase gel.

Cet exemple permet de montrer que le gel reste efficace sur une large amplitude de conditions de température. Que ce soit à haute ou basse température, les supports sont globalement très bien contaminés, et ce en partant d'une contamination initiale dépassant le 10⁶ CFU dans la plupart des cas.

**Tableau 3 : Efficacité du gel biocide sur des agents pathogènes pour des températures extrêmes.**

| Agent | Support | Température (°C) | Contamination initiale du support (CFU) | Contamination finale du support (CFU) | Contamination résiduelle des paillettes (CFU) |
|---|---|---|---|---|---|
| *B. anthracis* (spores) | Acier inoxydable | 5 | 9,3.10⁶ | < I.d. | <I.d. |
| | | 50 | 6,3.10⁶ | < I.d. | 72 |
| | Céramique RATP | 5 | 3,2.10⁶ | < I.d. | <I.d. |
| | | 50 | 2,4.10⁶ | 5 | 276 |
| *Y. pestis* (bactéries) | Acier inoxydable | 5 | 3,5.10⁵ | <I.d. | <I.d. |
| | | 50 | 2,8.10³ | <I.d. | <I.d. |
| | Céramique RATP | 5 | 8,9.10⁴ | <I.d. | <I.d. |
| | | 50 | 1,6.10⁴ | <I.d. | <I.d. |

### Exemple 15 :

Dans cet exemple, on montre l'efficacité du gel biocide selon l'invention sur Y.p. dans des conditions aggravées de salissure des supports. En d'autres termes, dans cet exemple, on montre que le gel de décontamination biologique GB79 selon l'invention, est efficace sur des supports souillés. Le protocole d'essai est similaire à celui de l'exemple 13, à l'exception des supports qui sont préalablement souillés au pinceau par un mélange de 1% d'argile de type montmorillonite, 10% d'huile de moteur 10W40 et 89% d'éthanol, et ce avant l'application du contaminant sur sa surface. Seule la contamination à la peste est testée.

Les résultats sont présentés dans le Tableau 4. On constate que l'effet dégraissant et décontaminant du gel selon l'invention est suffisant pour éliminer souillure et contamination biologique bactérienne sur le support.

**Tableau 4 : Efficacité du gel biocide sur des agents pathogènes pour des supports souillés.**

| Agent | Support | Température (°C) | Contamination initiale du support | Contamination finale du support | Contamination résiduelle des paillettes |
|---|---|---|---|---|---|
| *Y. pestis* (bactéries) | Acier inoxydable souillé | ∼20 | 7,9.10⁶ | <I.d. | <I.d. |
| | Céramique RATP souillée | ∼20 | 1,6.10⁷ | <I.d. | <I.d. |

### Conclusion de tous les exemples:

Au regard des exemples 12 à 15 mais aussi 1 à 11, il apparaît que le gel alcalin et oxydant de décontamination biologique selon l'invention, est un outil efficace de lutte contre des contaminants biologiques pathogènes présents sur différentes infrastructures suite à une dissémination biologique accidentelle ou malveillante.

### Annexe 1.

### Protocole de test de cytotoxicité :

Le test de cytotoxicité utilisé est illustré sur les Figures 20 et 21. Les cellules humaines HeLa sont cultivées à 37°C dans une atmosphère contenant 5% de CO₂ sur des flasques de culture de 150 cm² dans du milieu DMEM (Dulbecco's Modified Eagle Medium) contenant 100 U/mL de pénicilline et 100 µg/mL de streptomycine.

Les cellules sont ensemencées à une densité de 50,000 cellules par puits dans des plaques 96 puits à fond en scintillant solide Cytostar-T (Perkin-Elmer). Les cellules (150 µL dans du DMEM complet: DMEM + 10% de sérum de veau foetal, SVF) sont ajoutées dans chaque puits de la microplaque. Le milieu complet complémenté de toxine (50 µL) est ensuite ajouté à chaque puits. En règle générale, une concentration différente de ricine par ligne est utilisée. Après une incubation de 20 h, le milieu (200 µL) est éliminé et remplacé par un milieu DMEM sans leucine (Eurobio) contenant 10% de SVF et 0,5 µCi/mL de 14C-leucine (GE). Après une incubation de 6 h à 37°C, l'incorporation de radioactivité par les cellules est déterminée par lecture des plaques par un compteur à scintillation Wallac 1450 microbeta trilux (PE) (Figure 20).

Comme ces toxines bloquent la synthèse des protéines, les cellules affectées ne sont plus capables d'incorporer la leucine radiomarquée. Par contre, les cellules non traitées à la ricine ou avec des concentrations très faibles de ricine (10⁻¹⁴-10⁻¹⁶ M) synthétisent toujours des protéines et incorporent donc l'acide aminé radiomarqué. Comme les cellules concentrent le radioélément suffisamment près du fond du puits, cela entraîne une excitation du scintillant contenu dans les plaques et conduit à l'émission de photons détectée par le compteur à scintillation (mesure en coups par minutes, cpm). Ces données sont ensuite exprimées en pourcentage de synthèse de protéine par les cellules. Les courbes de cytotoxicité peuvent ainsi être tracées et l'EC50 déterminé (Figure 21).

### REFERENCES.

[1] CUER F., FAURE S. « Gel de décontamination biologique et procédé de décontamination de surfaces utilisant ce gel », FR-A1-2962046 et WO-A1-2012/001046.
[2] HOFFMAN D., Mc GUIRE R. "Oxidizer gels for detoxification of chemical and biological agents", US-B1-6,455,751.
[3] HARPER B., LARSEN L. "A comparison of decontamination technologies for biological agents on selected commercial surface materials", Biological weapons improved response program, April 2001.
[4] FAURE S., FOURNEL B., FUENTES P., LALLOT Y. "Procédé de traitement d'une surface par un gel de traitement, et gel de traitement", FR-A1-2 827 530.
[5] FAURE S., FUENTES P., LALLOT Y. "Gel aspirable pour la décontamination de surfaces et utilisation", FR-A1-2 891470.

## Revendications

1. Gel de décontamination biologique, constitué par une solution colloïdale comprenant :
- 5% à 30% en masse, de préférence 5% à 25% en masse, de préférence encore 8% à 20% en masse par rapport à la masse du gel, d'au moins un agent viscosant inorganique ;
- un agent actif de décontamination biologique constitué par la combinaison d'une base minérale choisie parmi les hydroxydes de métaux alcalins, les hydroxydes de métaux alcalinoterreux, et leurs mélanges, et d'un agent oxydant stable en milieu basique choisi parmi les permanganates, les persulfates, l'ozone, les hypochlorites, et leurs mélanges; la base minérale étant présente à raison de 0,05 à 10 mol/L de gel, de préférence à raison de 0,1 à 5 mol/L de gel, et l'agent oxydant stable en milieu basique étant présent à raison de 0,05 à 5 mol/L de gel, de préférence de 0,1 à 2 mol/L de gel;
- éventuellement 0,1% à 2% en masse par rapport à la masse du gel, d'au moins un agent tensio-actif ;
- et le reste de solvant ;
**caractérisé en ce que** le gel ne contient pas de polymère super-absorbant.

2. Gel selon la revendication 1, dans lequel la base minérale est choisie parmi l'hydroxyde de sodium, l'hydroxyde de potassium, et leurs mélanges, et l'agent oxydant stable en milieu basique est choisi parmi les hypochlorites, et leurs mélanges; de préférence, l'agent actif de décontamination biologique est constitué par la combinaison de soude et d'hypochlorite de sodium.

3. Gel selon l'une quelconque des revendications précédentes, dans lequel l'agent viscosant inorganique est choisi parmi les oxydes de métaux tels que les alumines, les oxydes de metalloïdes à l'exception de la silice, les hydroxydes de métaux, les hydroxydes de metalloïdes, les oxyhydroxydes de métaux, les oxyhydroxydes de metalloïdes, les aluminosilicates, les argiles telles que la smectite, et leurs mélanges ; de préférence, l'agent viscosant inorganique est constitué par une ou plusieurs alumine(s) ; de préférence encore la ou les alumine(s) représente(nt) de 5% à 30% en masse, de préférence de 8% à 17% en masse par rapport à la masse totale du gel.

4. Gel selon l'une quelconque des revendications précédentes, dans lequel l'agent tensio-actif est choisi parmi les agents tensio-actifs non ioniques tels que les copolymères blocs, séquences comme les copolymères séquencés d'oxyde d'éthylène et d'oxyde de propylène, et les acides gras éthoxylés; et leurs mélanges ; et/ou dans lequel le solvant est choisi parmi l'eau, les solvants organiques et leurs mélanges.

5. Gel selon l'une quelconque des revendications précédentes, qui comprend en outre au moins un pigment minéral.

6. Procédé de décontamination biologique d'une surface d'un substrat solide contaminée par au moins une espèce biologique se trouvant sur ladite surface, dans lequel on réalise au moins un cycle comprenant les étapes successives suivantes :
a) on applique le gel selon l'une quelconque des revendications 1 à 5 sur ladite surface ;
b) on maintient le gel sur la surface au moins pendant une durée suffisante pour que le gel détruise et/ou inactive et/ou absorbe l'espèce biologique, et pour que le gel sèche et forme un résidu sec et solide non pulvérulent contenant éventuellement ladite espèce biologique;
c) on élimine le résidu sec et solide contenant éventuellement ladite espèce biologique.

7. Procédé selon la revendication 6, dans lequel le substrat est en au moins un matériau choisi parmi les métaux et alliages comme l'acier inoxydable; les aciers peints ; les polymères tels que les matières plastiques ou les caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments ; les mortiers et bétons; les plâtres ; les briques; la pierre naturelle ou artificielle ; les céramiques.

8. Procédé selon l'une quelconque des revendications 6 et 7, dans lequel l'espèce biologique est choisie parmi les bactéries, les champignons, les levures, les virus, les toxines, les spores, les prions et les protozoaires ; de préférence l'espèce biologique est choisie parmi les espèces bio-toxiques telles que les spores pathogènes comme par exemple les spores de *Bacillus anthracis,* les toxines comme par exemple la toxine botulique ou la ricine, les bactéries comme les bactéries *Yersinia pestis* et les virus comme le virus de la vaccine ou les virus des fièvres hémorragiques par exemple de type Ebola.

9. Procédé selon l'une quelconque des revendications 6 à 8, dans lequel le gel est appliqué sur la surface à raison de 100 g à 2000 g de gel par m² de surface, de préférence de 500 g à 1500 g de gel par m², de préférence encore de 600 g à 1000 g de gel par m² de surface.

10. Procédé selon l'une quelconque des revendications 6 à 9, dans lequel le gel est appliqué sur la surface solide par pulvérisation, au pinceau, ou avec une taloche.

11. Procédé selon l'une quelconque des revendications 6 à 10, dans lequel lors de l'étape b), le séchage est réalisé à une température de 1°C à 50°C, de préférence de 15°C à 25°C, et sous une humidité relative de 20% à 80%, de préférence de 20% à 70%.

12. Procédé selon l'une quelconque des revendications 6 à 11, dans lequel le gel est maintenu sur la surface pendant une durée de 2 à 72 heures, de préférence de 2 à 48 heures, de préférence encore de 3 à 24 heures.

13. Procédé selon l'une quelconque des revendications 6 à 12, dans lequel le résidu sec et solide se présente sous la forme de particules, par exemple de paillettes, d'une taille de 1 à 10 mm, de préférence de 2 à 5 mm.

14. Procédé selon l'une quelconque des revendications 6 à 13, dans lequel le résidu sec et solide est éliminé de la surface solide par brossage et/ou aspiration.

15. Procédé selon l'une quelconque des revendications 6 à 14, dans lequel le cycle décrit est répété de 1 à 10 fois en utilisant le même gel lors de tous les cycles ou en utilisant des gels différents lors d'un ou de plusieurs cycle(s).

16. Procédé selon l'une quelconque des revendications 6 à 15, dans lequel, lors de l'étape b), le gel, avant séchage total, est remouillé avec une solution d'un agent de décontamination biologique, de préférence avec la solution de l'agent actif biologique du gel appliqué lors de l'étape a) dans le solvant de ce gel.

## Patentansprüche

1. Bio-Dekontaminationsgel gebildet bestehend aus einer kolloidalen Lösung, umfassend :
- einen auf die Masse des Gels bezogenen Massenanteil von 5% bis 30%, vorzugsweise 5% bis 25%, noch einmal vorzugsweise 8% bis 20% von wenigstens einem anorganischen viskosmachenden Mittel;
- ein aktives biologisches Dekontaminationsmittel, gebildet bestehend der aus Kombination einer mineralischen Base, ausgewählt unter den Alkalimetallhydroxiden, den Erdalkalimetallhydroxiden, und ihren Mischungen, und einem im basischen Milieu stabilen Oxidationsmittel, ausgewählt unter den Permanganaten, den Persulfaten, dem Ozon, den Hypochloriten, und ihren Mischungen, wobei die mineralische Base mit 0,05 bis 10 mol/Liter Gel, vorzugsweise mit 0,1 bis 5 mol/Liter Gel präsent ist, und das im basischen Milieu stabile Oxidationsmittel mit 0,05 bis 5 mol/Liter Gel, vorzugsweise 0,1 bis 2 mol/Liter Gel präsent ist;
- eventuell, wenigstens ein oberflächenaktives Mittel, mit einem Massenanteil von 0,1 % bis 2% in Bezug auf die Masse des Gels;
- und der Rest Lösungsmittel;
**dadurch gekennzeichnet, dass** das Gel kein superabsorbierendes Polymer enthält.

2. Gel nach Anspruch 1, bei dem die mineralische Base ausgewählt wird unter dem Natriumhydroxid, dem Kaliumhydroxid, und ihren Mischungen, und das im basischen Milieu stabile Oxidationsmittel ausgewählt wird unter den Hypochloriten, und ihren Mischungen, wobei das aktive biologische Dekontaminationsmittel vorzugsweise aus der Kombination von Soda und Natriumhyperchlorit besteht.

3. Gel nach einem der vorhergehenden Ansprüche, bei dem das anorganische viskosmachende Mittel ausgewählt wird unter den Metalloxiden wie zum Beispiel den Aluminiumoxiden, den Metalloidoxiden, ausgenommen das Siliciumdioxid, den Metallhydroxiden, den Metalloidhydroxiden, den Metalloxyhydroxiden, den Metalloidoxyhydroxiden, den Aluminiumsilikaten, den Tonen, zum Beispiel Smektit, und ihren Mischungen, wobei das anorganische viskosmachende Mittel vorzugsweise aus einer (mehrere) Aluminiumoxid(e) besteht; noch einmal vorzugsweise stellt (stellen) das (die) Aluminiumoxid(e) einen Massenanteil von 5% bis 30%, vorzugsweise einen Massenanteil von 8% bis 17% in Bezug auf die Gesamtmasse des Gels dar.

4. Gel nach einem der vorhergehenden Ansprüche, bei dem das oberflächenaktive Mittel ausgewählt wird unter den nichtionischen oberflächenaktiven Mitteln wie etwa den Block-, sequenzierten, Copolymeren, wie die ethylenoxid- und propylenoxidsequenzierten Copolymere, und den ethoxilierten Fettsäuren ; und ihren Mischungen ; und/oder bei denen das Lösungsmittel ausgewählt wird unter Wasser, den organischen Lösungsmitteln und ihren Mischungen.

5. Gel nach einem der vorhergehenden Ansprüche, das außerdem mindestens ein mineralisches Pigment enthält.

6. Verfahren zur biologischen Dekontamination einer Oberfläche eines Feststoff-Substrats, kontaminiert durch wenigstens eine auf der genannten Oberfläche befindliche biologische Spezies, bei dem man wenigstens einen die folgenden sukzessiven Schritte umfassenden Zyklus realisiert:
a) man bringt das Gel nach einem der Ansprüche 1 bis 5 auf die genannte ober fläche auf;
b) man belässt das Gel während einer ausreichenden Dauer auf der Oberfläche, so dass die biologische Spezies durch das Gel zerstört und/oder inaktiviert und/oder absorbiert wird und dass das Gel trocknet und einen trockenen und festen, nichtpulvrigen Rückstand bildet, der eventuell die biologische Spezies enthält;
c) man entfernt den eventuell die genannte biologische Spezies enthaltenden Rückstand.

7. Verfahren nach Anspruch 6, bei dem das Substrat aus wenigstens einem Material ist, ausgewählt unter den Metallen und Legierungen wie etwa dem rostfreiem Stahl; den lackierten Stählen ; den Polymeren wie zum Beispiel den Kunststoffen oder den Kautschuken wie Polyvinylchlorid oder PVC, den Polypropylenen oder PP, den Polyethylenen oder PE, insbesondere den Hochdichte-Polyethylenen HDPE, den Polymethacrylaten oder PMMA, den Polyvinylidenfluoriden oder PVDF, den Polycarbonaten oder PC ; den Gläsern ; den Zementen ; den Mörteln und Betonen ; den Gipsen ; den Ziegelsteinen ; den Natur- oder Kunststeinen ; den Keramiken.

8. Verfahren nach einem der Ansprüche 6 und 7, bei dem die biologische Spezies ausgewählt wird unten den Bakterien, den Pilzen, den Hefen, den Viren, den Toxinen, den Sporen, den Prionen und den Protozoonen ; vorzugsweise wird die biologische Spezies ausgewählt unter den biotoxischen Spezies wie etwa den pathogenen Sporen wie zum Beispiel den *Bacillus anthracis*-Sporen, den Toxinen wir zum Beispiel dem Botulinumtoxin oder dem Ricin, den Bakterien wie den *Yersinia pestis*-Bakterien und den Viren wie dem Vakzine-Virus oder den Viren der hämorrhagischen Fieber, zum Beispiel des Typs Ebola.

9. Verfahren nach einem der Ansprüche 6 bis 8, bei dem das Aufbringen des Gels auf die Oberfläche realisiert wird mit 100 g bis 2000 g Gel pro m² Oberfläche, bevorzugt 500 g bis 1500 g Gel pro m² Oberfläche, noch einmal bevorzugt 600 g bis 1000 g Gel pro m² Oberfläche.

10. Verfahren nach einem der Ansprüche 6 bis 9, bei dem das Gel auf die feste Oberfläche durch Zerstäubung, mit dem Pinsel, oder mit einer Traufel aufgebracht wird.

11. Verfahren nach einem der Ansprüche 6 bis 10, bei dem während des Schritts b) die Trocknung bei einer Temperatur von 1°C bis 50°C, vorzugsweise von 15°C bis 25°C, und bei einer relativen Luftfeuchtigkeit von 20% bis 80%, vorzugsweise von 20% bis 70% stattfindet.

12. Verfahren nach einem der Ansprüche 6 bis 11, bei dem das Gel während einer Dauer von 2 bis 72 Stunden, vorzugsweise 2 bis 48 Stunden, noch vorzugsweise 3 bis 24 Stunden auf der Oberfläche belassen wird.

13. Verfahren nach einem der Ansprüche 6 bis 12, bei dem sich der trockene und feste Rückstand in Form von Partikeln präsentiert, zum Beispiel Plättchen mit einer Größe von 1 bis 10 mm, vorzugsweise von 2 bis 5 mm.

14. Verfahren nach einem der Ansprüche 6 bis 13, bei dem der trockene und feste Rückstand durch Bürsten und/oder Absaugen von der festen Oberfläche entfernt wird.

15. Verfahren nach einem der Ansprüche 6 bis 14, bei dem der beschriebene Zyklus 1 bis 10 Mal wiederholt wird, wobei für alle Zyklen das gleiche Gel benutzt wird, oder indem für einen Zyklus oder für mehrere Zyklen unterschiedliche Gels benutzt werden.

16. Verfahren nach einem der Ansprüche 6 bis 15, bei dem in Schritt b) das Gel vor der totalen Trocknung wieder angefeuchtet bzw. nass gemacht wird mit einer Lösung eines biologischen Dekontaminationsmittels, vorzugsweise mit der Lösung des aktiven biologischen Mittels des in Schritt a) aufgebrachten Gels in dem Lösungsmittel dieses Gels.

## Claims

1. A biological decontamination gel, consisting of a colloidal solution comprising:
- 5% to 30% by mass, preferably 5% to 25% by mass, still preferably 8% to 20% by mass based on the mass of the gel, of at least one inorganic viscosifying agent;
- an active biological decontamination agent consisting of the combination of a mineral base selected from hydroxides of alkaline metals, hydroxides of earth alkaline metals, and mixtures thereof, and of an oxidizing agent stable in a basic medium selected from permanganates, persulfates, ozone, hypochlorites, and mixtures thereof; the mineral base being present in an amount from 0.05 to 10 mol/L of gel, preferably in an amount from 0.1 to 5 mol/L of gel, and the oxidizing agent stable in a basic medium being present in an amount from 0.05 to 5 mol/L of gel, preferably from 0.1 to 2 mol/L of gel;
- optionally 0.1% to 2% by mass based on the mass of the gel, of at least one surfactant;
- and the balance of solvent;
**characterized in that** the gel does not contain any super-absorbent polymer.

2. The gel according to claim 1, wherein the mineral base is selected from sodium hydroxide, potassium hydroxide, and mixtures thereof, and the oxidizing agent stable in a basic medium is selected from hypochlorites, and mixtures thereof; preferably the active biological decontamination agent consists of the combination of soda and sodium hypochlorite.

3. The gel according to any one of the preceding claims, wherein the inorganic viscosifying agent is selected from oxides of metals such as aluminas, oxides of metalloids except for silica, hydroxides of metals, hydroxides of metalloids, oxyhydroxides of metals, oxyhydroxides of metalloids, aluminosilicates, clays such as smectite, and mixtures thereof; preferably the inorganic viscosifying agent consists of one or several alumina(s); more preferably the alumina(s) represent(s) from 5% to 30% by mass, preferably from 8% to 17% by mass based on the total mass of the gel.

4. The gel according to any one of the preceding claims, wherein the surfactant is selected from the non-ionic surfactants such as sequenced, block copolymers, such as block copolymers of ethylene oxide and propylene oxide, and ethoxylated fatty acids; and mixtures thereof; and/or wherein the solvent is selected from water, organic solvents and mixtures thereof.

5. The gel according to any one of the preceding claims, which further comprises at least one mineral pigment.

6. A method for biological decontamination of a surface of a solid substrate contaminated with at least one biological species found on said surface, wherein at least one cycle is carried out comprising the following successive steps:
a) the gel according to any one of claims 1 to 5 is applied on said surface;
b) the gel is maintained on the surface for at least a sufficient duration so that the gel destroys and/or inactivates and/or absorbs the biological species, and so that the gel dries and forms a dry and non-powdered solid residue possibly containing said biological species;
c) the dry and solid residue possibly containing said biological species is removed (eliminated).

7. The method according to claim 6, wherein the substrate is made of at least one material selected from metals and alloys like stainless steel; painted steels; polymers such as plastics or rubbers like poly(vinyl chloride)s or PVC, polypropylenes or PP, polyethylenes or PE notably the high density polyethylenes or HDPE, poly(methyl methacrylate)s or PMMA, poly(vinylidene fluoride)s or PVDF, polycarbonates or PC; glasses; cements; mortars and concretes; plasters; bricks; natural or artificial stone; ceramics.

8. The method according to any one of claims 6 and 7, wherein the biological species is selected from among bacteria, fungi, yeasts, viruses, toxins, spores, prions and protozoa; preferably the biological species is selected from bio-toxic species such as pathogenic spores like for example the spores of *Bacillus anthracis,* toxins such as for example botulinic toxin or ricin, bacteria like bacteria *Yersinia pestis* and viruses like the virus of vaccine or the virus of hemorrhagic fevers for example of the Ebola type.

9. The method according to any one of claims 6 to 8, wherein the gel is applied on the surface in an amount from 100 g to 2,000 g of gel per m² of surface, preferably from 500 g to 1,500 g of gel per m², still preferably from 600 g to 1,000 g of gel per m² of surface.

10. The method according to any one of claims 6 to 9, wherein the gel is applied on the solid surface by spraying, with a brush, or with a trowel.

11. The method according to any one of claims 6 to 10, wherein during step b), the drying is carried out at a temperature from 1°C to 50°C, preferably from 15°C to 25°C, and under relative humidity from 20% to 80%, preferably from 20% to 70%.

12. The method according to any one of claims 6 to 11, wherein the gel is maintained on the surface for a period from 2 to 72 hours, preferably from 2 to 48 hours, more preferably from 3 to 24 hours.

13. The method according to any one of claims 6 to 12, wherein the dry and solid residue appears as particles, for example flakes, with a size from 1 to 10 mm, preferably from 2 to 5 mm.

14. The method according to any one of claims 6 to 13, wherein the dry and solid residue is removed from the solid surface by brushing and/or suction.

15. The method according to any one of claims 6 to 14, wherein the described cycle is repeated from 1 to 10 times by using the same gel during all the cycles or by using different gels during one or several cycle(s).

16. The method according to any one of claims 6 to 15, wherein, during step b), the gel, before total drying, is re-wetted with a solution of a biological decontamination agent, preferably with the solution of the active biological agent of the gel applied during step a) in the solvent of this gel.
